# EUROPEAN PATENT APPLICATION

(11) **EP 4 817 528 A1**
(43) Date of publication of application: **07.10.2026**
(21) Application number: 26162838.2
(22) Date of filing: 06.03.2026
(51) Int. Cl.: A61B 6/58

(54) **SYSTEMS AND METHODS FOR CT DETECTOR CALIBRATION USING A WIRE PHANTOM**

(30) Priority: 31.03.2025 US 202519096510
(71) Applicant: GE Precision Healthcare LLC, Waukesha, WI 53188 (US)
(72) Inventor: MCBROOM, Gary V., Waukesha, 53188-1696 (US); DELONG SAMALIK, Michelle Marie Severino, Waukesha, 53188-1696 (US); EVANGELIST, Matthew J., Waukesha, 53188-1696 (US); CEDERSTRÖM, Björn, 10691 Stockholm (SE); KIM, Changlyong, Waukesha, 53188-1696 (US); KULAS, Ronald George, Waukesha, 53188-1696 (US); LIANG, Jingyi, Waukesha, 53188-1696 (US); ADAMAK, Mark, Waukesha, 53188-1696 (US); LING, Jiayin, Waukesha, 53188-1696 (US)
(74) Representative: Kilburn & Strode LLP

(57) **Abstract**

Systems and methods are provided for calibrating a computed tomography (CT) system (100) using a wire phantom (302) coupled to a table of the CT system (100), where during a calibration of the CT system (100), a position of a detector element (202) of a detector array of the CT system (100) is measured using a wire (304) of the wire phantom (302), during a rotational scan performed using the CT system (100). During a subsequent scan, the measured position of the detector element (202) may be applied during reconstruction of an image from projection data acquired via the CT system (100). The method relies on a straightness of the wire (304) within the wire phantom (302), which is maintained by spherical swage-end terminations (1012, 1014) of the wire (304) when tension is applied to the wire (304) A phantom holder (323) of the wire phantom (302) may include a clamp (1701) and a handle (2002) for positioning the wire phantom (302) on the table.

## Description

### CROSS-REFERENCE TO RELATED APPLICATIONS

The present application is a continuation-in-part of U.S. Non-Provisional Application No. 18/313,820, entitled "SYSTEMS AND METHODS FOR CT DETECTOR CALIBRATION USING A WIRE PHANTOM," and filed on May 8, 2023. The entire contents of the above-listed application are hereby incorporated by reference for all purposes.

### TECHNICAL FIELD

Embodiments of the subject matter disclosed herein relate to computed tomography (CT) imaging systems, and in particular, calibration of CT imaging systems using a wire phantom.

### BACKGROUND

In computed tomography (CT) imaging systems, an electron beam generated by a cathode is directed towards a target within an x-ray tube. A fan-shaped or cone-shaped beam of x-rays produced by electrons colliding with the target is directed towards a subject, such as a patient. After being attenuated by the object, the x-rays impinge upon an array of radiation detector elements. An electrical signal is generated at each detector element, and the electrical signals generated at the detector elements are used to reconstruct an image of the object, where each electrical signal corresponds to a voxel/pixel of the image.

A quality of the image in terms of resolution, contrast-to-noise ratio, and other factors may depend on an alignment of each detector element within the detector arrays. Misalignments of the detector elements may increase a number of artifacts in the image and/or reduce the quality of the image. The misalignments may be measured and corrected using mechanical alignment techniques. The mechanical alignment techniques may use several kinds of measurement methods, such as using positioning tools with contact and/or laser. Based on measurement data, the detector elements can be realigned using a set of mechanical tools. However, current capabilities for alignment may not be feasible for newer versions of CT systems including smaller detector elements, which may require less than 10um alignment accuracy.

### SUMMARY

The current disclosure at least partially addresses one or more of the above identified issues by a computed tomography (CT) system, comprising a processor and a non-transitory memory including instructions that when executed, cause the processor to perform a first series of rotational scans of a wire phantom to determine a straightness of a wire of the wire phantom; perform a second series of rotational scans of the wire phantom to determine an angle of inclination of the wire; in response to the straightness of the wire being within a first threshold deviation and the angle of inclination of the wire being within a second threshold deviation: during a calibration scan performed using the CT system: measure a position of each detector element of a detector array of the CT system with respect to a location of the wire; and during a subsequent scan performed using the CT system, apply the measured position of each detector element during reconstruction of an image from projection data acquired via the CT system; and display the reconstructed image on a display device of the CT system; wherein the straightness of the wire is maintained within the first threshold deviation by a tension on the wire between a first spherical swage-end termination of the wire seated in a free-floating termination part coupled to a first end cap at a first end of a tube of the wire phantom via a spring, and a second spherical swage-end termination seated in a hollow threaded portion of a bolt bolted to center point of an inner circular surface of a second end cap of the tube at a second, opposite end of the wire phantom.

The above advantages and other advantages, and features of the present description will be readily apparent from the following Detailed Description when taken alone or in connection with the accompanying drawings. It should be understood that the summary above is provided to introduce in simplified form a selection of concepts that are further described in the detailed description. It is not meant to identify key or essential features of the claimed subject matter, the scope of which is defined uniquely by the claims that follow the detailed description. Furthermore, the claimed subject matter is not limited to implementations that solve any disadvantages noted above or in any part of this disclosure.

### BRIEF DESCRIPTION OF THE DRAWINGS

Various aspects of this disclosure may be better understood upon reading the following detailed description and upon reference to the drawings in which:
FIG. 1 shows a pictorial view of a CT imaging system, in accordance with one or more embodiments of the present disclosure;
FIG. 2 shows a block schematic diagram of an exemplary CT imaging system, in accordance with one or more embodiments of the present disclosure;
FIG. 3A is a wire phantom for calibrating a CT imaging system, in accordance with one or more embodiments of the present disclosure;
FIG. 3B shows an alignment of the wire phantom of FIG. 3A with respect to a table of a CT system, in accordance with one or more embodiments of the present disclosure;
FIG. 3C shows an alignment of the wire phantom of FIG. 3A with respect to a detector array of a CT system, in accordance with one or more embodiments of the present disclosure;
FIG. 4 is a schematic diagram of an exemplary detector array of a CT system, in accordance with one or more embodiments of the present disclosure;
FIG. 5A is a flowchart illustrating an exemplary method for increasing an accuracy of a CT image using calibration data, in accordance with one or more embodiments of the present disclosure;
FIG. 5B is a flowchart illustrating an exemplary method for calibrating a CT system using a wire phantom, in accordance with one or more embodiments of the present disclosure;
FIG. 6 is a schematic diagram showing an exemplary alignment of a wire phantom with respect to an x-ray source and an x-ray detector array, in accordance with one or more embodiments of the present disclosure;
FIG. 7 is a graph of a sinogram generated using a wire phantom, in accordance with one or more embodiments of the present disclosure;
FIG. 8 shows how a parabolic function may be fitted to an output of a detector array to determine a position of a detector element, in accordance with one or more embodiments of the present disclosure;
FIG. 9 shows an exemplary projection geometry used to calculate a position of a detector element of a detector array with respect to a wire phantom, in accordance with one or more embodiments of the present disclosure;
FIG. 10 shows an exemplary embodiment of the wire phantom including self-centering wire terminations;
FIG. 11 shows a perspective view of a first self-centering wire termination of the embodiment of FIG. 10;
FIG. 12 shows a spherical swage-end of the first self-centering termination;
FIG. 13 shows an expanded view of the spherical swage-end;
FIG. 14 shows an end cap of the first self-centering termination;
FIG. 15 shows a perspective view of a second self-centering wire termination of the embodiment of FIG. 10;
FIG. 16 shows a base-end fitting of the second self-centering wire termination;
FIG. 17 shows a phantom holder of the wire phantom including a clamp;
FIG. 18 shows a first set of components of the clamp of FIG. 17;
FIG. 19 shows a second set of components of the clamp of FIG. 17; and
FIG. 20 shows a handle of the phantom holder of FIG. 17.

The drawings illustrate specific aspects of the described systems and methods. Together with the following description, the drawings demonstrate and explain the structures, methods, and principles described herein. In the drawings, the size of components may be exaggerated or otherwise modified for clarity. Well-known structures, materials, or operations are not shown or described in detail to avoid obscuring aspects of the described components, systems and methods.

### DETAILED DESCRIPTION

This description and embodiments of the subject matter disclosed herein relate to methods and systems for increasing a quality of images acquired via a computed tomography (CT) system. Typically, in computed tomography (CT) imaging systems, an x-ray source emits a fan-shaped beam or a cone-shaped beam towards an object, such as a patient. X-rays emitted by the x-ray source are attenuated to varying degrees by the object prior to being detected by radiation detector elements arranged in one or more detector arrays. In CT systems, the x-ray source and the detector arrays are generally rotated about a gantry within an imaging plane and around the patient, and images are generated from projection data at a plurality of views at different view angles. For example, for one rotation of the x-ray source, 1000 views may be generated by the CT system. The beam, after being attenuated by the patient, impinges upon the array of radiation detector elements. The detector array typically includes a collimator for collimating x-ray beams received at the detector, a scintillator disposed adjacent to the collimator for converting x-rays to light energy, and photodiodes for receiving the light energy from the adjacent scintillator and producing electrical signals therefrom. An intensity of the attenuated beam radiation received at the detector array is typically dependent upon the attenuation of the x-ray beam by the patient. Each detector element of a detector array produces a separate electrical signal indicative of the attenuated beam received by each detector element. The electrical signals are transmitted to a data processing system for analysis. The data processing system processes the electrical signals to facilitate generation of an image.

The detector arrays may be curved, such that the detector elements arranged on the detector arrays are aligned towards the x-ray source, to more accurately and efficiently detect the x-rays emitted by the x-ray source. The detector elements may be organized in clusters. For example, a detector array may include a plurality of detector modules; each detector module may include a plurality of detector element groupings called chiclets; and each chiclet may include a plurality of detector elements.

The quality of an image reconstructed from projection data collected from the detector elements may be dependent on an alignment of each detector element with the x-ray source. Some detector elements may not be precisely aligned during the assembly due to the limited capability of a mechanical alignment tool or may become misaligned over time or due to a use of the CT system. For example, a detector module of a detector array may be damaged, and the detector module may be replaced with a new detector module. The new detector module may not be accurately aligned due to a lack of a high precision alignment tool at a customer site. To begin with, , detector modules may not be assembled accurately within the detector array; chiclets may be misaligned within a detector module; and/or detector elements may be misaligned within a chiclet.

Misalignments in a number of detector elements may increase a number of noise artifacts in the image and/or reduce the quality of the image. The image quality may be increased by correcting for the misalignments. To correct for the misalignments, the CT system may be periodically calibrated, where positions of the detector modules, chiclets, or detector elements may be mechanically adjusted. However, measuring and correcting the misalignments is currently performed using alignment techniques that may not be feasible for newer versions of CT systems having smaller detector elements and pixel sizes.

To address the problem of calibrating CT systems including the smaller detector elements and correcting for misalignments of the detector elements, a method is proposed herein for measuring the position of detector elements directly that does not rely on the mechanical alignment techniques, but rather generates the detector positions from projection data collected using a wire phantom. Calibration vectors may be generated from the measured detector positions, which may be applied to projection data acquired during a subsequent scan during image reconstruction, to correct for misaligned detector positions. In some embodiments, rather than correcting for misaligned detector positions, the measured detector positions may be directly applied during image reconstruction instead of target design detector positions.

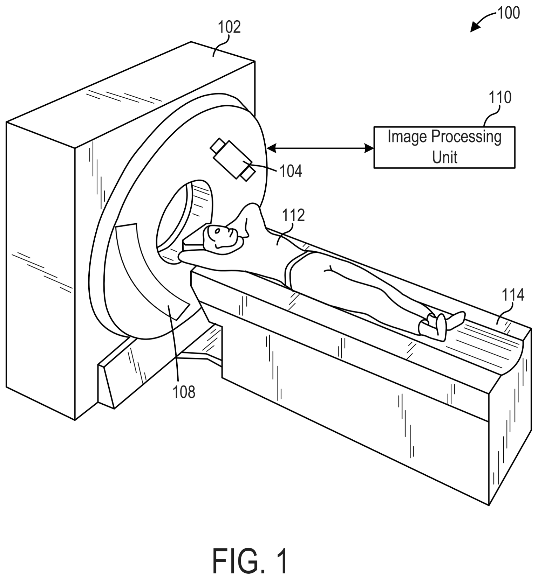

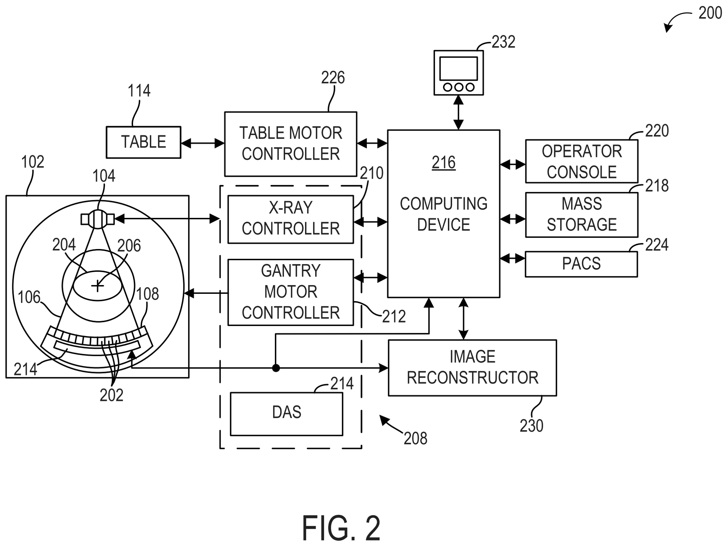

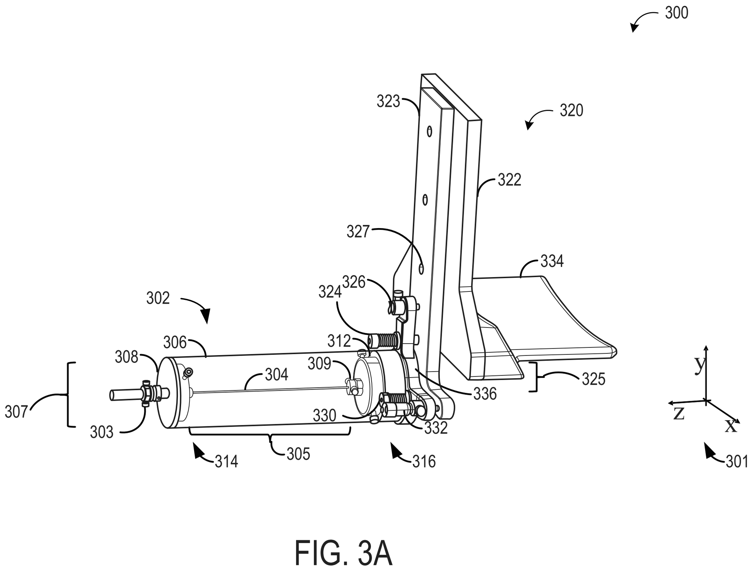

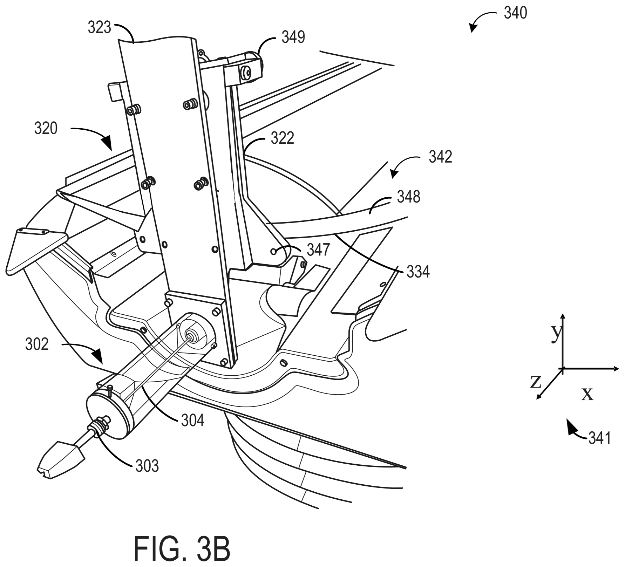

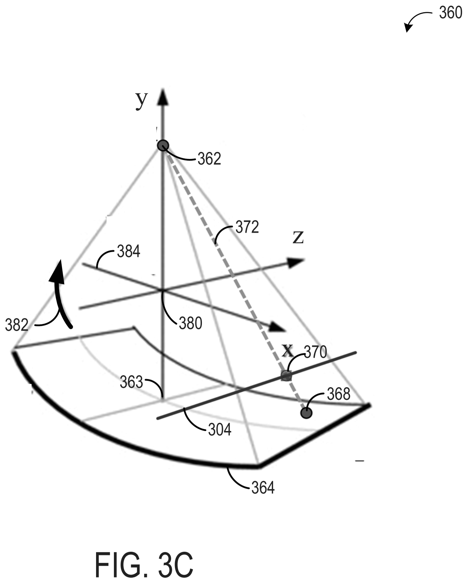

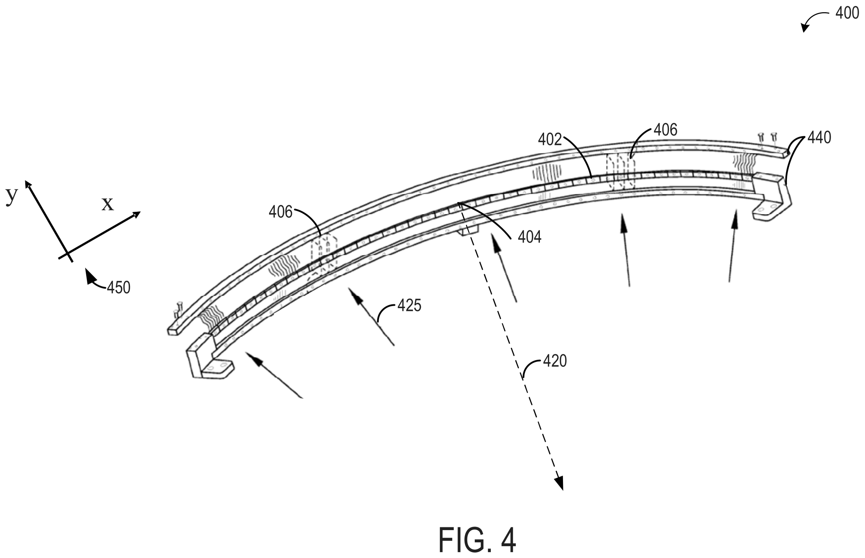

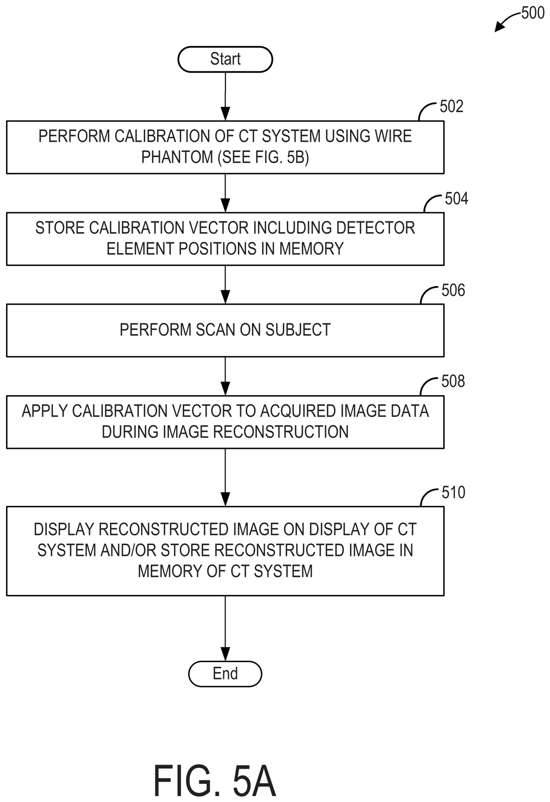

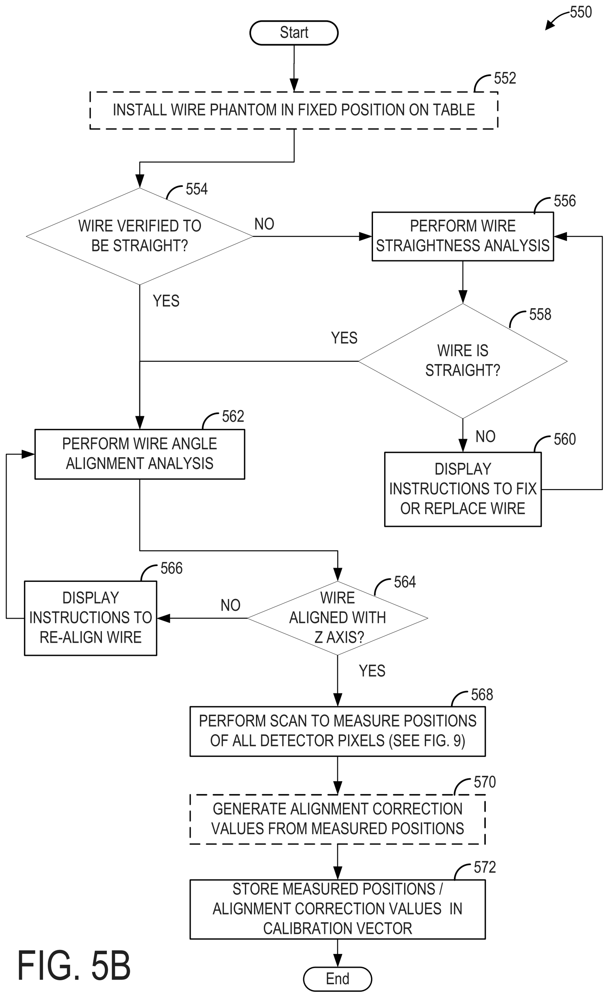

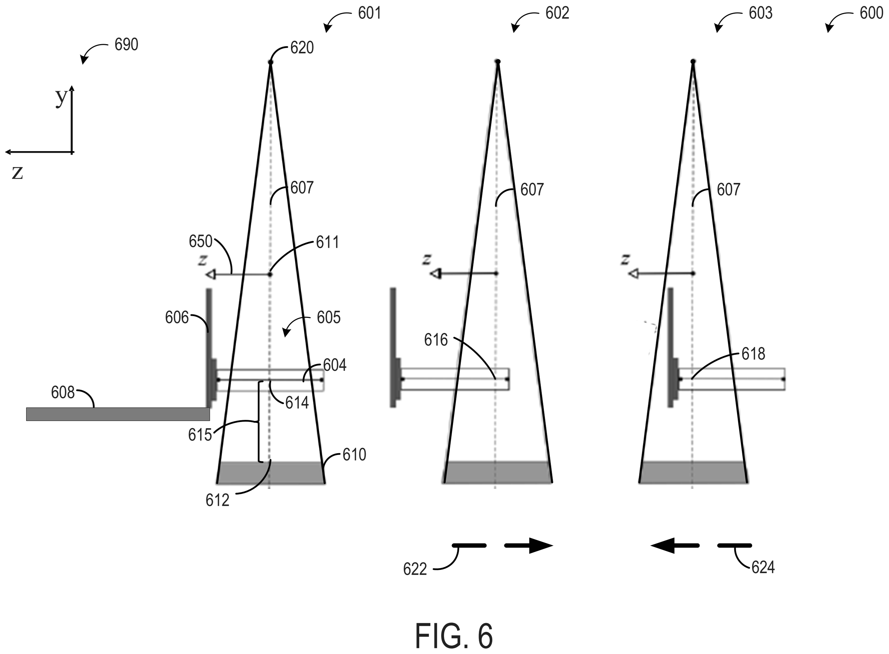

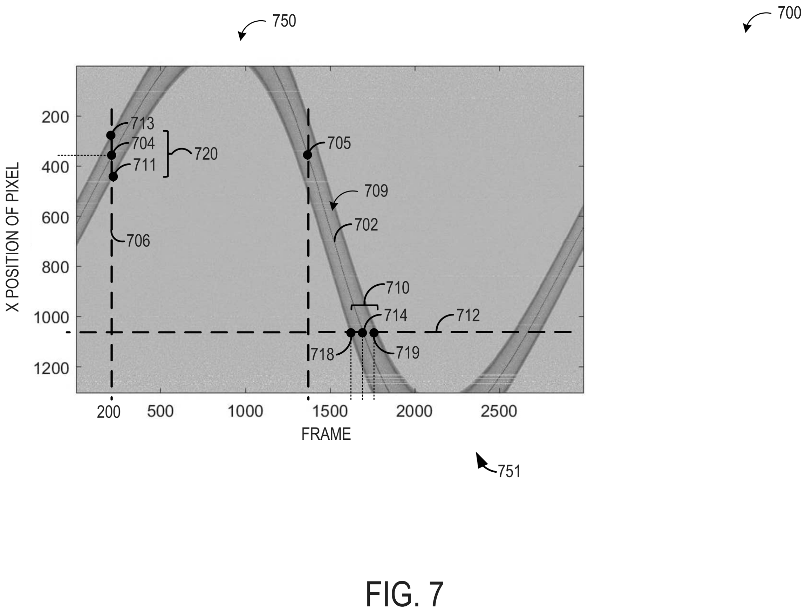

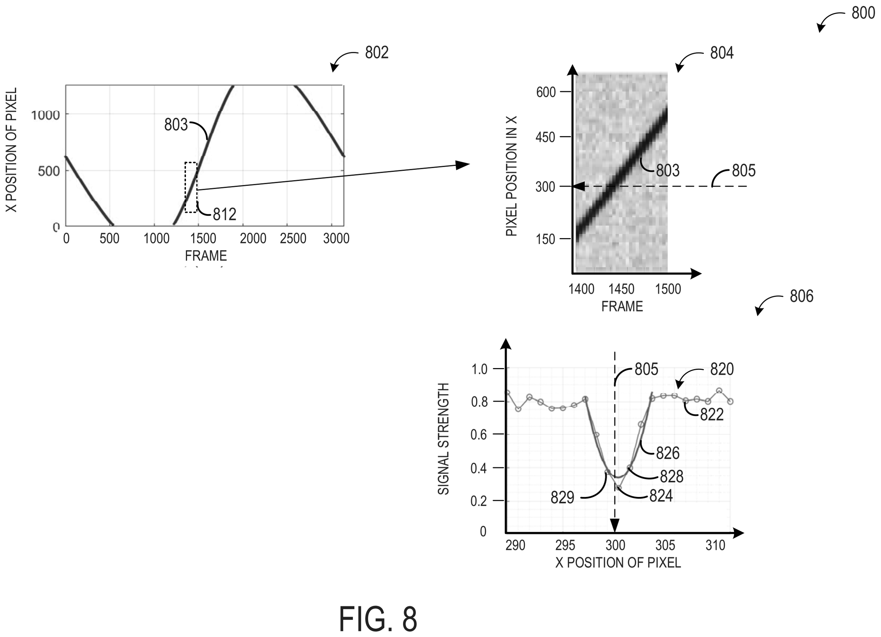

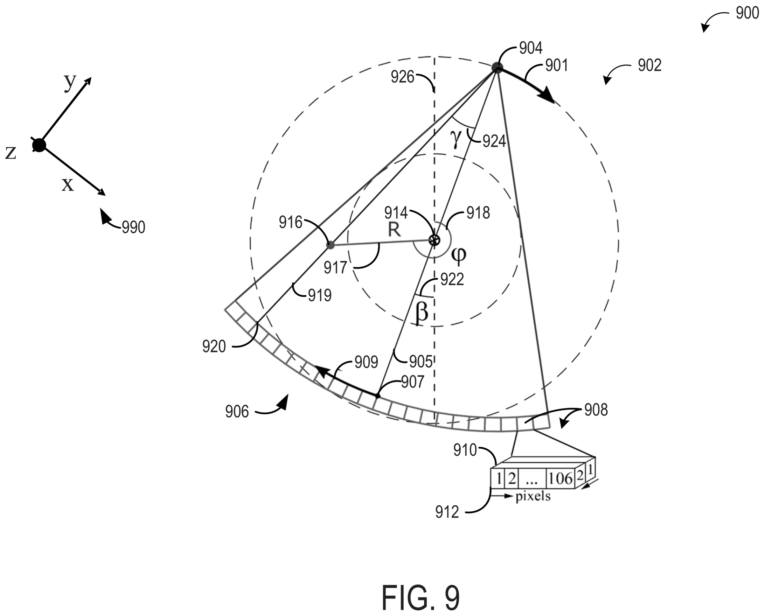

An example of a CT system is provided in FIGS. 1 and 2. FIG. 3A shows an exemplary wire phantom that may be used to calibrate the CT system. The wire phantom may be mounted on a table of the CT system, as depicted in FIG. 3B. FIG. 3C shows a position of a wire of the wire phantom with respect to an x-ray source and a detector array of the CT system during a calibration process described herein. The detector array may include a plurality of detector elements organized in modules, as shown in FIG. 4. A method for calibrating the CT system to correct for misalignments of the detector elements is shown in FIG. 5B, and a method for applying measured detector locations generated during the calibration process during image reconstruction to increase a quality of a resulting image is shown in FIG. 5A. FIG. 6 shows a positioning of the wire phantom within an imaging plane of the CT system during the calibration. During the calibration, projection data may be collected at the detector elements as a gantry holding the detector array and an x-ray source rotates around the wire phantom. Due to the rotation, the projection data collected during calibration may be represented graphically as a sinogram, as shown in FIG. 7, which may indicate which detector elements detect an attenuation of x-rays by the wire at a given view angle or frame. The attenuation may be greatest at a detector element indicated by a parabolic function fitted to detector output data, as shown in FIG. 8. A position of the detector element with respect to the wire may be measured in accordance with the projection geometry shown in FIG. 9.

FIGS. 3A, 3B, 4, and 6 show example configurations with relative positioning of the various components. If shown directly contacting each other, or directly coupled, then such elements may be referred to as directly contacting or directly coupled, respectively, at least in one example. Similarly, elements shown contiguous or adjacent to one another may be contiguous or adjacent to each other, respectively, at least in one example. As an example, components laying in face-sharing contact with each other may be referred to as in face-sharing contact. As another example, elements positioned apart from each other with only a space there-between and no other components may be referred to as such, in at least one example. As yet another example, elements shown above/below/underneath one another, at opposite sides to one another, or to the left/right of one another may be referred to as such, relative to one another. Further, as shown in the figures, a topmost element or point of element may be referred to as a "top" of the component and a bottommost element or point of the element may be referred to as a "bottom" of the component, in at least one example. As used herein, top/bottom, upper/lower, above/below, may be relative to a vertical axis of the figures and used to describe positioning of elements of the figures relative to one another. As such, elements shown above other elements are positioned vertically above the other elements, in one example. As yet another example, shapes of the elements depicted within the figures may be referred to as having those shapes (e.g., such as being circular, straight, planar, curved, rounded, chamfered, angled, or the like). Further, elements shown intersecting one another may be referred to as intersecting elements or intersecting one another, in at least one example. Further still, an element shown within another element or shown outside of another element may be referred as such, in one example.

FIG. 1 illustrates an exemplary CT system 100 configured for CT imaging. Particularly, CT system 100 is configured to image a subject 112 such as a patient, an inanimate object, one or more manufactured parts, and/or foreign objects such as dental implants, stents, and/or contrast agents present within the body. In one embodiment, the CT system 100 includes a gantry 102, which in turn, may further include at least one x-ray source 104 configured to project a beam of x-ray radiation 106 (see FIG. 2) for use in imaging the subject 112 laying on a table 114. Specifically, the x-ray source 104 is configured to project the x-ray radiation beams 106 towards a detector array 108 positioned on the opposite side of the gantry 102. Although FIG. 1 depicts a single x-ray source 104, in certain embodiments, multiple x-ray sources and detectors may be employed to project a plurality of x-ray radiation beams for acquiring projection data at different energy levels corresponding to the patient. In some embodiments, the x-ray source 104 may enable rapid peak kilovoltage (kVp) switching. In the embodiments described herein, the x-ray detector employed is a photon-counting detector which is capable of differentiating x-ray photons of different energies.

In certain embodiments, the CT system 100 further includes an image processing unit 110 configured to reconstruct images of a target volume of the subject 112 using an iterative or analytic image reconstruction method. For example, the image processing unit 110 may use an analytic image reconstruction approach such as filtered back projection (FBP) to reconstruct images of a target volume of the patient. As another example, the image processing unit 110 may use an iterative image reconstruction approach such as advanced statistical iterative reconstruction (ASIR), conjugate gradient (CG), maximum likelihood expectation maximization (MLEM), model-based iterative reconstruction (MBIR), and so on to reconstruct images of a target volume of the subject 112. As described further herein, in some examples the image processing unit 110 may use both an analytic image reconstruction approach such as FBP in addition to an iterative image reconstruction approach.

In some CT imaging system configurations, an x-ray source projects a cone-shaped x-ray radiation beam which is collimated to lie within an X-Y-Z plane of a Cartesian coordinate system and generally referred to as an "imaging plane." The x-ray radiation beam passes through an object being imaged, such as the patient or subject. The x-ray radiation beam, after being attenuated by the object, impinges upon an array of detector elements. The intensity of the attenuated x-ray radiation beam received at the detector array is dependent upon the attenuation of an x-ray radiation beam by the object. Each detector element of the array produces a separate electrical signal that is a measurement of the x-ray beam attenuation at the detector location. The attenuation measurements from all the detector elements are acquired separately to produce a transmission profile.

In some CT systems, the x-ray source and the detector array are rotated with a gantry within the imaging plane and around the object to be imaged such that an angle at which the x-ray beam intersects the object constantly changes. A group of x-ray radiation attenuation measurements, e.g., projection data, from the detector array at one gantry angle is referred to as a "view." A "scan" of the object includes a set of views made at different gantry angles, or view angles, during one revolution of the x-ray source and detector.

FIG. 2 illustrates an exemplary imaging system 200 similar to the CT system 100 of FIG. 1. In accordance with aspects of the present disclosure, the imaging system 200 is configured for imaging a subject 204 (e.g., the subject 112 of FIG. 1). In one embodiment, the imaging system 200 includes the detector array 108 (see FIG. 1). The detector array 108 further includes a plurality of detector elements 202 that together sense the x-ray radiation beam 106 (see FIG. 2) that pass through the subject 204 (such as a patient) to acquire corresponding projection data. In some embodiments, the detector array 108 may be fabricated in a multi-slice configuration including the plurality of rows of cells or detector elements 202, where one or more additional rows of the detector elements 202 are arranged in a parallel configuration for acquiring the projection data. An exemplary detector array configuration is described in greater detail below in reference to FIG. 4.

In certain embodiments, the imaging system 200 is configured to traverse different angular positions around the subject 204 for acquiring desired projection data. Accordingly, the gantry 102 and the components mounted thereon may be configured to rotate about a center of rotation 206 for acquiring the projection data, for example, at different energy levels. Alternatively, in embodiments where a projection angle relative to the subject 204 varies as a function of time, the mounted components may be configured to move along a general curve rather than along a segment of a circle.

As the x-ray source 104 and the detector array 108 rotate, the detector array 108 collects data of the attenuated x-ray beams. The data collected by the detector array 108 undergoes pre-processing and calibration to condition the data to represent the line integrals of the attenuation coefficients of the scanned subject 204. The processed data are commonly called projections. In some examples, the individual detectors or detector elements 202 of the detector array 108 may include photon-counting detectors which register the interactions of individual photons into one or more energy bins.

The acquired sets of projection data may be used for basis material decomposition (BMD). During BMD, the measured projections are converted to a set of material-density projections. The material-density projections may be reconstructed to form a pair or a set of material-density map or image of each respective basis material, such as bone, soft tissue, and/or contrast agent maps. The density maps or images may be, in turn, associated to form a 3D volumetric image of the basis material, for example, bone, soft tissue, and/or contrast agent, in the imaged volume.

Once reconstructed, the basis material image produced by the imaging system 200 reveals internal features of the subject 204, expressed in the densities of two basis materials. The density image may be displayed to show these features. In traditional approaches to diagnosis of medical conditions, such as disease states, and more generally of medical events, a radiologist or physician would consider a hard copy or display of the density image to discern characteristic features of interest. Such features might include lesions, sizes and shapes of particular anatomies or organs, and other features that would be discernable in the image based upon the skill and knowledge of the individual practitioner.

In one embodiment, the imaging system 200 includes a control mechanism 208 to control movement of the components such as rotation of the gantry 102 and the operation of the x-ray source 104. In certain embodiments, the control mechanism 208 further includes an x-ray controller 210 configured to provide power and timing signals to the x-ray source 104. Additionally, the control mechanism 208 includes a gantry motor controller 212 configured to control a rotational speed and/or position of the gantry 102 based on imaging requirements.

In certain embodiments, the control mechanism 208 further includes a data acquisition system (DAS) 214 configured to sample analog data received from the detector elements 202 and convert the analog data to digital signals for subsequent processing. The DAS 214 may be further configured to selectively aggregate analog data from a subset of the detector elements 202 into so-called macro-detectors, as described further herein. The data sampled and digitized by the DAS 214 is transmitted to a computer or computing device 216. It is noted that the computing device 216 may be the same or similar to image processing unit 110, in at least one example. In one example, the computing device 216 stores the data in a storage device or mass storage 218. The storage device 218, for example, may be any type of non-transitory memory and may include a hard disk drive, a floppy disk drive, a compact disk-read/write (CD-R/W) drive, a Digital Versatile Disc (DVD) drive, a flash drive, and/or a solid-state storage drive.

Additionally, the computing device 216 provides commands and parameters to one or more of the DAS 214, the x-ray controller 210, and the gantry motor controller 212 for controlling system operations such as data acquisition and/or processing. In certain embodiments, the computing device 216 controls system operations based on operator input. The computing device 216 receives the operator input, for example, including commands and/or scanning parameters via an operator console 220 operatively coupled to the computing device 216. The operator console 220 may include a keyboard (not shown) or a touchscreen to allow the operator to specify the commands and/or scanning parameters.

Although FIG. 2 illustrates one operator console 220, more than one operator console may be coupled to the imaging system 200, for example, for inputting or outputting system parameters, requesting examinations, plotting data, and/or viewing images. Further, in certain embodiments, the imaging system 200 may be coupled to multiple displays, printers, workstations, and/or similar devices located either locally or remotely, for example, within an institution or hospital, or in an entirely different location via one or more configurable wired and/or wireless networks such as the Internet and/or virtual private networks, wireless telephone networks, wireless local area networks, wired local area networks, wireless wide area networks, wired wide area networks, etc.

In one embodiment, for example, the imaging system 200 either includes, or is coupled to, a picture archiving and communications system (PACS) 224. In an exemplary implementation, the PACS 224 is further coupled to a remote system such as a radiology department information system, hospital information system, and/or to an internal or external network (not shown) to allow operators at different locations to supply commands and parameters and/or gain access to the image data.

The computing device 216 uses the operator-supplied and/or system-defined commands and parameters to operate a table motor controller 226, which in turn, may control a table 114 which may be a motorized table. Specifically, the table motor controller 226 may move the table 114 for appropriately positioning the subject 204 in the gantry 102 for acquiring projection data corresponding to the target volume of the subject 204.

As previously noted, the DAS 214 samples and digitizes the projection data acquired by the detector elements 202. Subsequently, an image reconstructor 230 uses the sampled and digitized x-ray data to perform high-speed reconstruction. Although FIG. 2 illustrates the image reconstructor 230 as a separate entity, in certain embodiments, the image reconstructor 230 may form part of the computing device 216. Alternatively, the image reconstructor 230 may be absent from the imaging system 200 and instead the computing device 216 may perform one or more functions of the image reconstructor 230. Moreover, the image reconstructor 230 may be located locally or remotely, and may be operatively connected to the imaging system 200 using a wired or wireless network. Particularly, one exemplary embodiment may use computing resources in a "cloud" network cluster for the image reconstructor 230.

In one embodiment, the image reconstructor 230 stores the images reconstructed in the storage device 218. Alternatively, the image reconstructor 230 may transmit the reconstructed images to the computing device 216 for generating useful patient information for diagnosis and evaluation. In certain embodiments, the computing device 216 may transmit the reconstructed images and/or the patient information to a display or display device 232 communicatively coupled to the computing device 216 and/or the image reconstructor 230. In some embodiments, the reconstructed images may be transmitted from the computing device 216 or the image reconstructor 230 to the storage device 218 for short-term or long-term storage.

Detector array 108 may include a plurality of detector modules. Each detector module may include a plurality of chiclets, where a chiclet is a set of individual sensors or detector elements.

Referring briefly to FIG. 4, an exemplary detector array 400 is shown. It should be understood that detector array 400 may be configured in different sizes and/or shapes, such as square, rectangular, circular, or another shape. An actual field of view (FOV) of detector array 400 may be directly proportional to the size and shape of detector array 400. In the depicted embodiment, detector array 400 has a curvature, where detector elements of detector array 400 are aligned towards an x-ray source located at a fixed distance above a center point 404 of detector array 400 along a y axis as indicated by dashed line 420 and reference axes 450.

Detector array 400 includes rails 440 having collimating blades or plates 406 placed therebetween. Plates 406 are positioned to collimate x-rays 425 before such beams impinge upon a plurality of detector modules 402 of detector array 400, which may be arranged between the plates 406. Each detector module 402 may include a plurality of chiclets, and each chiclet may include a plurality of detector elements or pixels. As an example, detector array 400 may include 25 detector modules 402; each detector module 402 may include 16 chiclets; and each chiclet may include 12 detector elements. Thus, each detector module 402 includes 192 detector elements, and a total of 4,800 detector elements are included in detector array 400. In one example, the width of the chiclet is 8.74 mm, and each detector element has 100 pixels, each pixel with a width of 365um in X and a pitch of 765 microns in Z. The small pixel size of 365um requires a precise mechanical alignment that may be difficult to achieve even though a positioning of the detector element within each chiclet may be controlled by robot placement.

Returning to FIG. 2, because the reconstructed images are generated from electrical signals produced at each detector element 202, an accuracy and/or image quality of the reconstructed images may depend on detector elements 202 being aligned within the detector arrays. Each detector element 202 has a position within a respective detector array. Not all the detector elements 202 may be correctly positioned. If an actual position of a detector element 202 varies from a design target position (e.g., if the detector element is misaligned), the accuracy of the reconstructed images may decrease.

The detector elements 202 may be very small and difficult to align. For example, a variation in alignment of less than 10% (e.g., less than 36.5 microns) may be desired to achieve a threshold image quality. If a portion of detector elements 202 are misaligned with respect to either or both of the x axis and the z axis by more than 10%, a quality of the reconstructed images may decrease.

In some examples, the portion of detector elements 202 may be misaligned due to a manufacturing process, or as a result of CT imaging system 200 being transported from a factory to a hospital. The portion of detector elements 202 may also become misaligned when a detector module of a detector array 108 becomes damaged and/or is replaced with a new detector module. The misalignment may occur between detector modules (e.g., module-to-module alignment), or between portions of a detector module (e.g., chiclet-to-chiclet alignment), or between sensors of a chiclet.

To ensure that a threshold image quality is achieved, one or more detector arrays 108 of CT imaging system 200 may be periodically calibrated. For example, the one or more detector arrays 108 may be calibrated when CT imaging system 200 is first installed in a healthcare facility, or after a damaged module is replaced, or after a predetermined amount of time, or at a different time. During calibration, a position of each detector element 202 may be measured with respect to a reference position provided by a wire phantom. Slight variations in the positions of the detector elements with respect to the reference position may be detected, collected, and used to generate a calibration vector for each detector array 108. The calibration vector may include the measured positions of the detector elements, or correction values for the measured positions of each detector element 202 of the detector array. The calibration vector may be used to perform a mechanical alignment of various detector elements of a detector array. Additionally or alternatively, during image reconstruction, the calibration vector may be applied to projection data acquired from the detector elements 202 to correct for the variations in the positions of the detector elements 202. By correcting for the variations, the image quality may be increased.

FIG. 3A shows an exemplary wire phantom assembly 300 used to calibrate the one or more detector arrays 108. Wire phantom assembly 300 includes a wire phantom 302, and a table mount 320, which may be used to mount wire phantom 302 to a table (e.g., table 114) of a CT imaging system such as CT imaging system 200 of FIG. 2.

Table mount 320 includes a mounting surface 334 that may be coupled to a surface of the table, and a phantom holder 323 to which wire phantom 302 may be coupled at a desirable position. Phantom holder 323 may allow wire phantom 302 to be adjustably positioned at a distance 325 below a lower surface of the table. For example, distance 325 may be adjusted by bolting phantom holder 323 to table mount 320 at different locations 327 (e.g., holes in phantom holder 323). Table mount 320 may include one or more rubber shims 322 coupled to phantom holder 323, which may be aligned in face-sharing contact with an edge of the table. Rubber shims 322 may provide a tighter and firmer coupling of table mount 320 with the table. A base plate 336 of wire phantom 302 may be coupled to phantom holder 323 via one or more bolts, such as a first bolt 324 and a second bolt 330.

Referring briefly to FIG. 3B, a table mounting diagram 340 shows an alignment of wire phantom 302 and table mount 320 with a table 342 of the CT imaging system. A mounting surface 334 may be aligned with a lower surface 348 of table 342. For example, mounting surface 334 may be clamped to lower surface 348. An alignment of phantom holder 323 with respect to lower surface 348 of table 342 along an x axis of the CT imaging system (e.g., in accordance with reference axes 341) may be adjusted via a first tilt screw 347. An alignment of phantom holder 323 with respect to lower surface 348 of table 342 along a y axis of the CT imaging system may be adjusted via a second tilt screw 349.

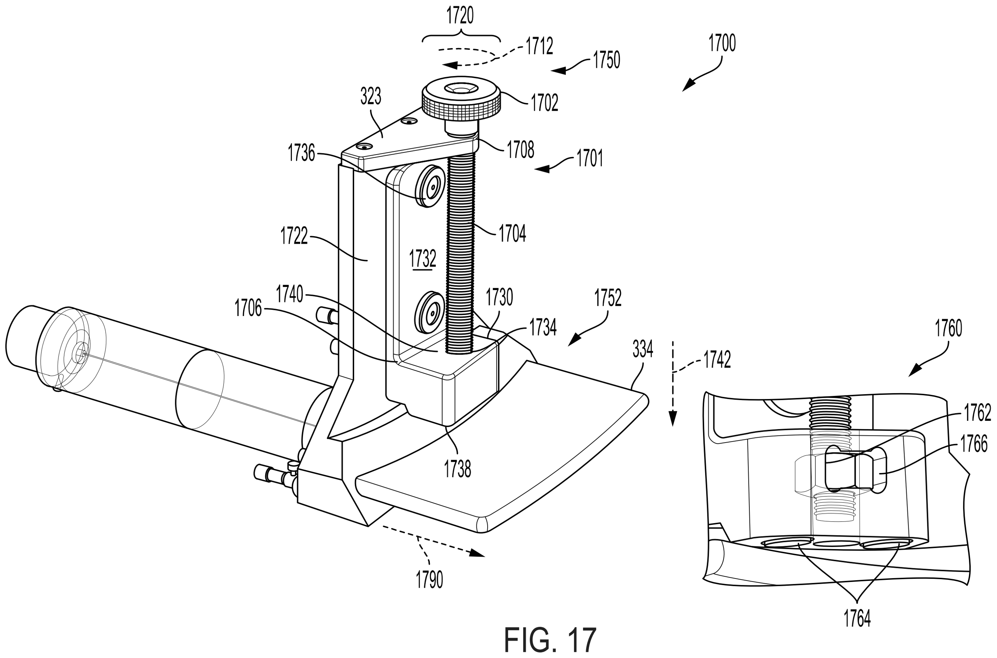

FIG. 17 shows a perspective view 1700 an example of phantom holder 323 including a clamp 1701 used to clamp mounting surface 334 to lower surface 348 of table 342 of FIG. 3B. Clamp 1701 includes a knob 1702 and a threaded portion 1704. Knob 1702 has a diameter 1720, which may be greater than a diameter of threaded portion 1704. An outer circumferential surface of knob 1702 may be textured to allow knob 1702 to be manually gripped and rotated by a user of wire phantom assembly 300.

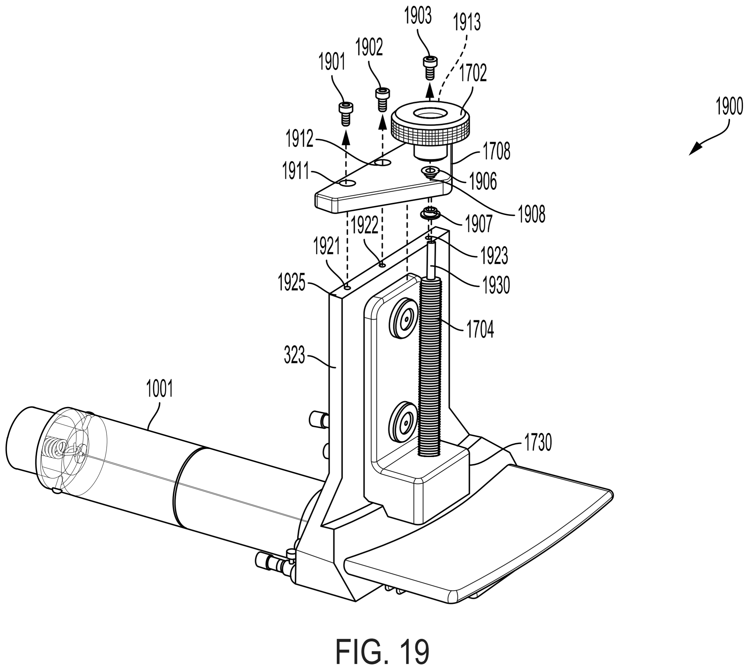

At a top side 1750 of clamp 1701, threaded portion 1704 extends through a hole in a bearing plate 1708 that is securely attached to an upper edge of phantom holder 323, as indicated in FIG. 19. Threaded portion 1704 extends through the hole down to a cradle 1706 at a bottom side 1752 of clamp 1701. Cradle 1706 may include a lower portion 1730 that extends outward from a back surface 1722 of phantom holder 323 towards table 342, in a direction indicated by an arrow 1790. Lower portion 1730 extends outward beyond a vertical position of threaded portion 1704 of clamp 1701, such that a bottom end 1734 of threaded portion 1704 may be inserted into lower portion 1730, as shown in an expanded view 1760. Bottom end 1734 of threaded portion 1704 may extend into a nut 1762 positioned in a cavity of lower portion 1730, which may be retained in place by one or more walls 1766 of the cavity. Cradle 1706 may be coupled to a back surface 1722 of phantom holder 323. Cradle 1706 may also include a flange portion 1732 that extends upward along back surface 1722, allowing cradle 1706 to be fastened to phantom holder 323 (back surface 1722) via one or more fasteners 1736. For example, fasteners 1736 may be bolts, screws, or a different type of fastener.

Cradle 1706 may be manufactured out of plastic or a similar material that may be flexibly deformed under pressure. Cradle 1706 may be positioned on phantom holder 323 such that phantom holder 323 may be mounted to table 342 by inserting mounting surface 334 into an accessory slot of table 342 such that a bottom surface 1738 of lower portion 1730 is in face sharing contact with an upper edge of table 342. A position of cradle 1706 on phantom holder 323 may be configured such that table 342 has a snug or interference fit between bottom surface 1738 and mounting surface 334. After mounting surface 334 has been inserted into the accessory slot of table 342, clamp 1701 may be actuated to increase a pressure between bottom surface 1738 of lower portion 1730 and the upper edge of table 342, by rotating knob 1702 of clamp 1701 clockwise, as indicated by an arrow 1712. An inner circumference of the hole in bearing plate 1708 through threaded portion 1704 is inserted may be threaded, such that as knob 1702 is rotated clockwise, bottom end 1734 of threaded portion 1704 engages with threads of nut 1762 to generate a downward force on lower portion 1730. As a result of the force, lower portion 1730 may be pushed against the upper surface of table 342, in a direction indicated by an arrow 1742. As can be seen in expanded view 1760 of lower portion 1730, bottom surface 1738 may include one or more pads 1764, which may protect the upper surface of table 342 from damage and increase a grip between lower portion 1730 and table 342. Thus, by rotating knob 1702, phantom holder 323 may be securely fastened to table 342. Phantom holder 323 may be removed from table 342 by rotating knob 1702 in an opposite, counterclockwise direction to engage nut 1762 in a reverse direction to release the pressure on table 342 exerted by the force, and sliding phantom holder 323 away from table 342.

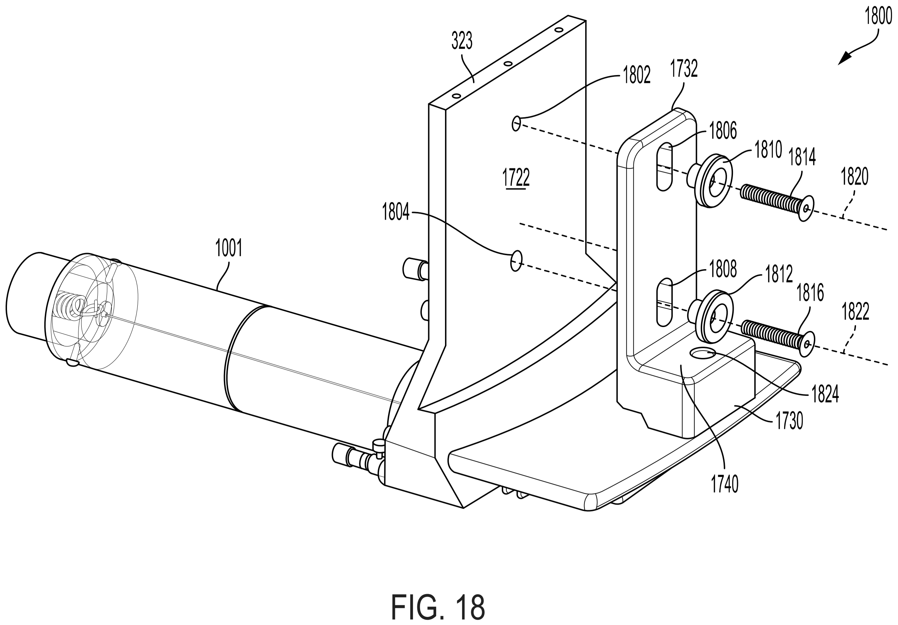

FIG. 18 shows an exploded view 1800 of cradle 1706. Back surface 1722 may include one or more holes, such as a hole 1802 and a hole 1804, into which cradle 1706 may be bolted. Flange portion 1732 of cradle 1706 includes holes 1806 and 1808, which may have an oblong, larger shape than holes 1802 and 1804 to allow cradle 1706 to be adjusted vertically on back surface 1722 after a preliminary attachment. Flange portion 1732 of cradle 1706 may be bolted to back surface 1722 via a first bolt 1814, which may be inserted into hole 1806 and hole 1802 along an axis 1820, and a second bolt 1816, which may be inserted into hole 1808 and hole 1804 along an axis 1822. A first guide 1810 may be included between first bolt 1814 and hole 1806, and a second guide 1812 may be included between second bolt 1816 and hole 1808. In some embodiments, upper flat surface 1740 of lower portion 1730 may have a recessed portion 1824 into which bottom end 1734 of threaded portion 1704 of clamp 1701 is seated when force is applied on lower portion 1730 by clamp 1701.

FIG. 19 shows a perspective view 1900 of an exemplary attachment of bearing plate 1708 to a top edge 1925 of phantom holder 323. In the depicted example, bearing plate 1708 is coupled to top edge 1925 via a plurality of screws, such as a screw 1901, a screw 1902, and a screw 1903. Screws 1901, 1902, and 1903 may extend through a hole 1911, a hole 1912, and a hole 1913 (obscured by knob 1702 in FIG. 19), respectively, in bearing plate 1708, and further into a hole 1921, a hole 1922, and a hole 1923, respectively, in top edge 1925 of phantom holder 323. Threaded portion 1704 of clamp 1701 may have an extending core 1930 that extends through a hole 1908 in bearing plate 1708 and into a center of knob 1702 of clamp 1701. A first bushing 1906 may be included between knob 1702 and hole 1908, and a second bushing 1907 may be included between hole 1908 and threaded portion 1704.

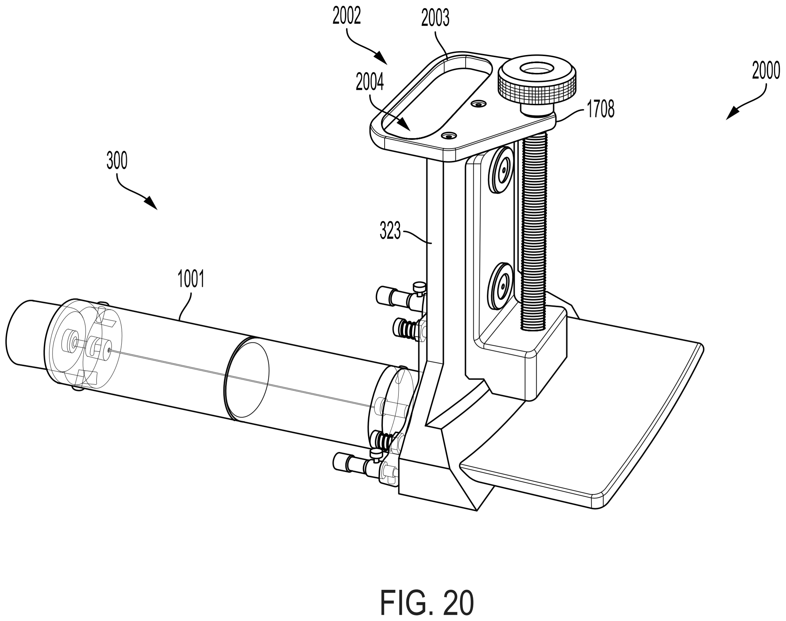

As another example, FIG. 20 shows an embodiment 2000 of phantom holder 323 in which bearing plate 1708 is extended to include a handle portion 2002 configured to allow wire phantom assembly 300 to be manually moved, carried, or inserted into a desirable position on table 342. Handle portion 2002 may include a hole 2004 into which a person's hand may be inserted to carry phantom holder 323, with an encircling portion 2003 around hole 2004. By including handle portion 2002 in bearing plate 1708, risks to wire phantom assembly 300 associated with handling and maneuvering wire phantom assembly 300 by tube 1001 may be reduced. Additionally, hole 2004 may be positioned on an opposite side of wire phantom assembly 300 from clamp 1701 and cradle 1706, such that a lift point of wire phantom assembly 300 is close to a center of gravity of wire phantom assembly 300, and wire phantom assembly 300 may remain level when lifted by handle portion 2002.

Returning to FIG. 3A, wire phantom 302 includes a wire 304 positioned along a central axis of a tube 306, where the central axis is aligned with a z axis of the CT imaging system. Tube 306 may protect wire 304 and provide a structure to maintain a straightness of wire 304 and protect the wire 302. Wire phantom 302 includes a first end cap 308 at a first side 314 (e.g., a gantry side) of wire phantom 302, and a second end cap 312 at a second side 316 (e.g., a table side) of wire phantom 302. Wire 304 extends from a first internal surface of first end cap 308 to a second internal surface of a second end cap 312. First end cap 308, second end cap 312, and tube 306 have a diameter 307. For example, diameter 307 may be 20 cm, or a different number.

Wire 304 may have a length 305, where length 305 is sufficiently long to cover a full detector array. For example, length 305 may be 150mm or longer. Wire 304 may include or be manufactured of a high attenuation material, such as steel or tungsten, which may attenuate x-rays and leave a distinctive signal in a readout of the CT imaging system during the scan. During calibration, a position of each detector element may be measured with respect to a position of wire 304. A scan may be performed on wire phantom 302. As a detector array rotates around the table via a gantry (e.g., gantry 102), an attenuation of x-rays by wire 304 may be detected by each detector element at different view angles.

A thickness or diameter of wire 304 may depend on a transaxial dimension of a detector element (e.g., a pixel). To measure each pixel location in a repeatable and reliable manner, a suitable diameter may be 2-4 times larger than the transaxial pixel dimension. In various embodiments, the suitable diameter is at least three times the transaxial dimension. In one embodiment, the transaxial dimension is 356 microns, and the diameter of wire 304 is 1mm (roughly three pixels wide). Thus, if the diameter of wire 304 is roughly equal to three pixel-widths, an attenuation of the x-rays by wire 304 during calibration may be detected by three or more pixels at a given view angle.

Wire 304 may be a multithread and/or braided wire, or a braided stainless steel cable, which may be easier to achieve a threshold straightness than a solid wire. For example, the threshold straightness may be a permitted variation of 30 microns from straight. In various embodiments, the threshold straightness may be maintained by placing wire 304 under a tension of a spring 309. Spring 309 may be positioned at first side 314 of wire phantom 302 (e.g., at first end cap 308), or spring 309 may be positioned at second side 316 of wire phantom 302 (e.g., at second end cap 312). Additionally, wire 304 may be molded with a plastic resin, which may further ensure that the threshold straightness is achieved. An accuracy of the positions of the detector elements measured during calibration may depend on a precise angle alignment of wire 304 with respect to the table and the gantry. In one example, the precise angle alignment may be within a threshold of 3 milliradians (mrad) of parallel to a central axis of the gantry. The precise angle alignment may be controlled and/or adjusted via one or more tilt screws positioned on phantom holder 323. Specifically, an alignment of wire 304 with respect to table mount 320 may be adjusted via a first wire phantom tilt screw 326 and/or a second wire phantom tilt screw 332. First wire phantom tilt screw 326 may adjust the vertical angle alignment of wire phantom 302 (e.g., in the y dimension indicated by references axes 301), and second wire phantom tilt screw 332 may adjust the horizontal angle alignment of wire phantom 302 (e.g., in the x dimension). Wire 304 may be aligned or re-aligned to parallel with the central axis of the gantry before or after each calibration and/or data analysis. The residual uncertainty coming from 3milliradians or less alignment accuracy may be corrected at a wire-itself adjustment and fitting step as a final step of the calibration.

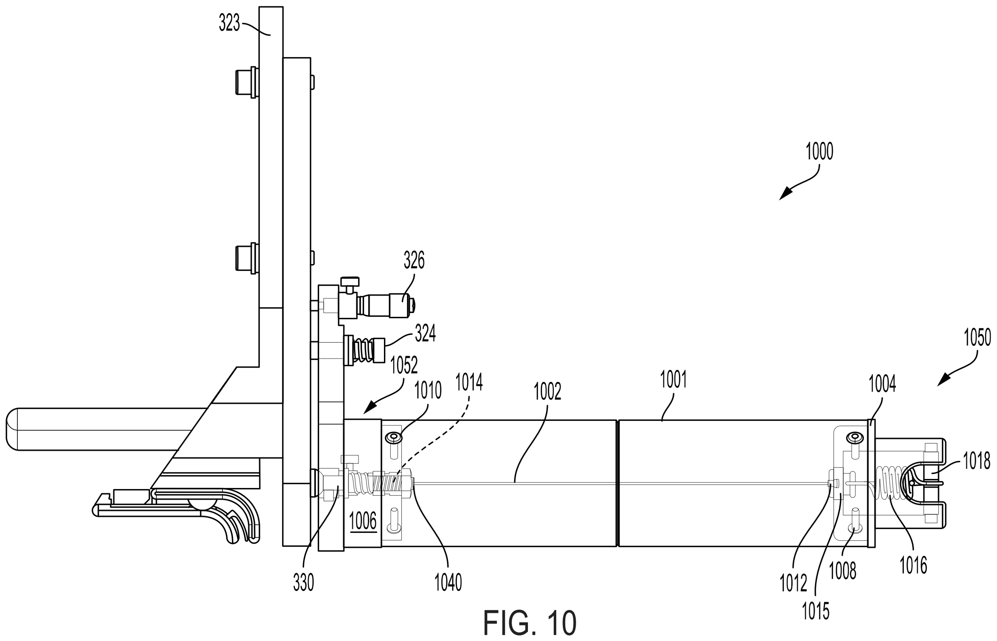

When spring 309 is positioned at end cap 308, because a termination 303 of wire 304 is outboard of spring 309, a tipping force may be imposed on end cap 308 when spring 309 is tensioned, that may impart a bend in wire 304. Over tightening of wire 304 (e.g., by a technician or user) may compress spring 309, and after end cap 308 bottoms, tension in wire 304 may increase rapidly, which may cause a soldered end of wire 304 pulling off at termination 303. To prevent over tightening of wire 304 while maintaining the straightness and/or tautness of wire 304, wire 304 may be configured with swage-end terminations that have spherical ends. The spherical ends may allow wire 304 to self-center within tube 306, thereby eliminating bending forces. The self-centering terminations are described in greater detail below in reference to FIGS. 10-17.

Referring now to FIG. 10, an embodiment 1000 of wire phantom 302 is shown, where embodiment 1000 includes a wire or cable 1002 (e.g., wire 304) with swage-end terminations with spherical ends. The spherical ends allow cable 1002 to self-center within a tube 1001 (e.g., tube 306), thereby preventing a bend in cable 1002 that may be caused by tightening cable 1002.

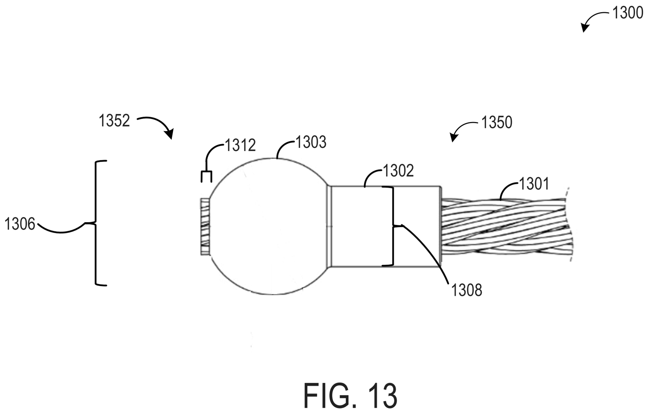

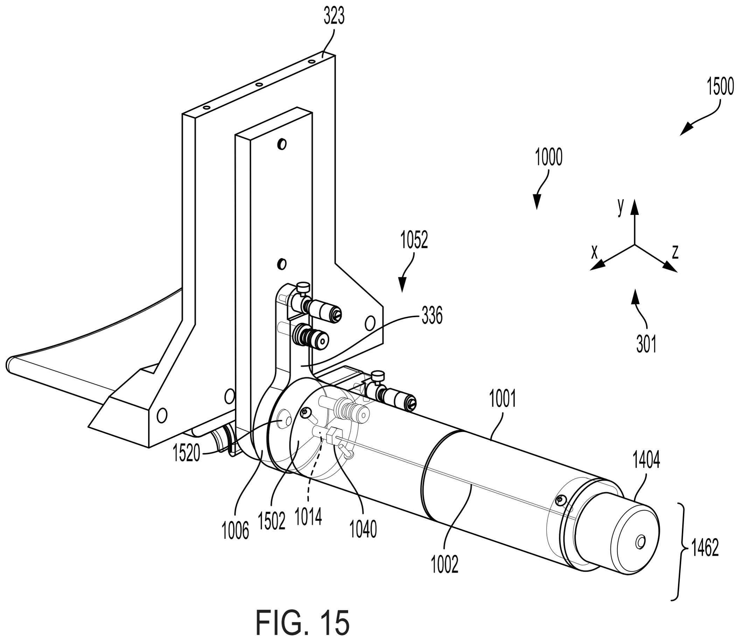

Tube 1001 includes a first end cap 1004 at a first end 1050 of tube 306, and a second end cap 1006 at a second end 1052 of tube 306. First end cap 1004 may be secured to tube 1001 via a first plurality of fasteners 1008 (e.g., screws, bolts, etc.) and second end cap 1006 may be secured to tube 1001 via a second plurality of fasteners 1010. Cable 1002 may be coupled to first end cap 1004 via a first spherical swage-end termination 1012, which may be seated in a termination part 1015 of first end cap 1004. Cable 1002 may be coupled to second end cap 1006 via a second spherical swage-end termination 1014, obscured in FIG. 10, which may be seated in a bolt 1014 coupled to second end cap 1006. A spherical swage-end termination such as first spherical swage-end termination 1012 and second spherical swage-end termination 1014 is shown in FIG. 13. Bolt 1014 and the second spherical swage-end termination are shown in greater detail in FIGS. 15-17.

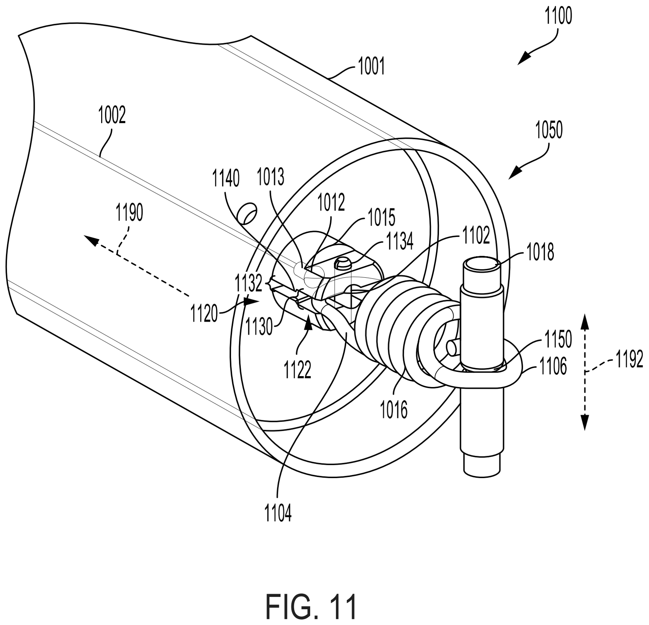

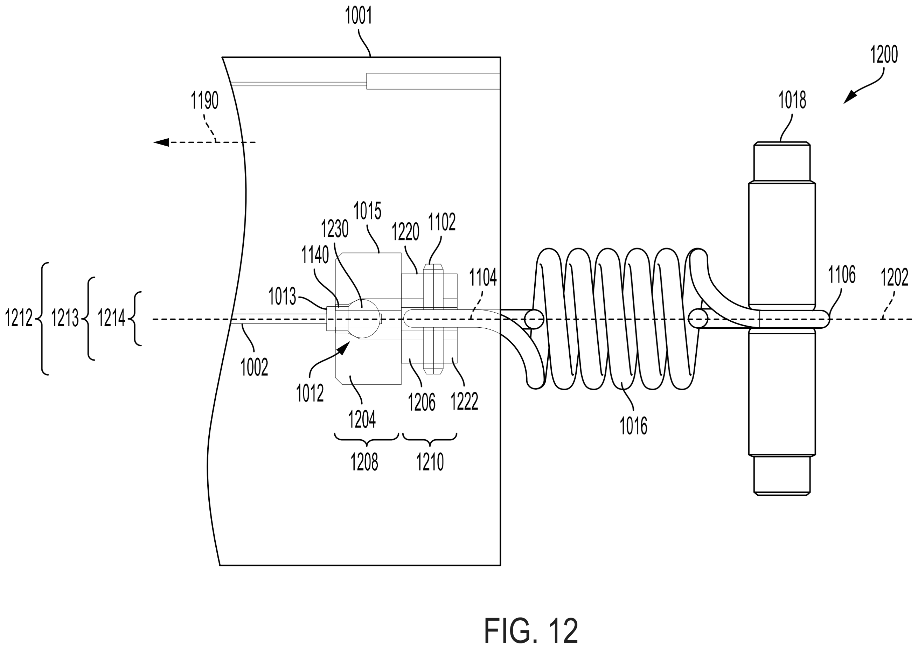

Termination part 1015 may be coupled to first end cap 1004 via a spring 1016. Termination part 1015 may be secured to a first end of spring 1016, and a second end of spring 1016 may be secured to first end cap 1004 via a pin 1018. The configuration of first spherical swage-end termination 1012, termination part 1015, and first end cap 1004 is shown in greater detail in FIGS. 11 and 12.

Turning first to FIG. 13, a spherical swage-end termination 1300 of a wire 1301 is shown, which may be a non-limiting example of first spherical swage-end termination 1012 and/or second spherical swage-end termination 1014 of wire 1002. In the depicted example, a wire 1301 is a braided stainless steel cable. In other examples, wire 1301 may comprise a different material or be configured differently, for example, in a non-braided manner. Spherical swage-end termination 1300 includes a shank 1302 at a first end 1350 of spherical swage-end termination 1300, and a spherical portion 1303 at a second end 1352 of spherical swage-end termination 1300. Wire 1301 may be inserted into shank 1302 and spherical portion 1303. In some embodiments, wire 1301 may protrude out from second end 1352 by a distance 1312. A first diameter 1306 of spherical portion 1303 is greater than a second diameter 1308 of shank 1302. As a result of first diameter 1306 being greater than second diameter 1308, spherical portion 1303 may be seated in a termination of an end cap of the wire phantom (e.g., termination part 1015 of first end cap 1004) when tension is applied to wire 1301, as shown in FIG. 11. An advantage of the swage-end terminations is that tension up to a full strength of wire 1301 may be supported (e.g., 160 lbf for braided 19-strand 300 series stainless steel cables).

Referring now to FIG. 11, a perspective view 1100 of first end 1050 is shown with an expanded view of first spherical swage-end termination 1012, termination part 1015, spring 1016, and pin 1018. In the depicted example, termination part 1015 has a cylindrical shape. In other examples, termination part 1015 may have a different shape. Termination part 1015 may include a slot 1120 in one side of termination part 1015 to allow first spherical swage-end termination 1012 to be seated into termination part 1015. Slot 1120 may include a wider portion 1122 at an end of termination part 1015 closest to first end 1050. A first width 1130 of wider portion 1122 may be greater than a diameter of first spherical swage-end termination 1012 (e.g., first diameter 1306). A second width 1132 of slot 1120 may be greater than a diameter of a shank 1013 (e.g., second diameter 1308), but less than the diameter of first spherical swage-end termination 1012. As a result, first spherical swage-end termination 1012 may be inserted into wider portion 1122 with wire 1002 and shank 1013 being simultaneously inserted into slot 1120 when there is no tension in wire 1002. A spherical portion of first spherical swage-end termination 1012 (e.g., spherical portion 1303) may be positioned at a center of termination part 1015 against a lip 1140 that prevents first spherical swage-end termination 1012 from moving in a direction 1190.

After first spherical swage-end termination 1012 is seated in termination part 1015, a first end coil 1104 of spring 1016 may be inserted into wider portion 1122 from first end 1050. A roll pin 1102 may then be inserted into a hole 1134 in termination part 1015 and threaded through first end coil 1104, coupling spring 1016 to termination part 1015. In some embodiments, hole 1134 may extend entirely through termination part 1015. In other embodiments, hole 1134 may not extend entirely through termination part 1015. Roll pin 1102 may be held in place by an interference fit, or a locking screw, in some examples. Pin 1018 may then be inserted into a second end coil 1106 of spring 1016, which may secure spring 1016 to first end cap 1004. Second end coil 1106 may be seated into a recessed circumferential portion 1150 of pin 1018, to prevent spring 1016 from sliding up or down pin 1018 in a direction indicated by bidirectional arrow 1192. When pin 1018 is secured to first end cap 1004, tension may be generated in wire 1002 by spring 1016. A material, a sizing, and a strength of spring 1016 may be configured to generate a desired amount of tension to maintain wire 1002 straight and centered in tube 1001.

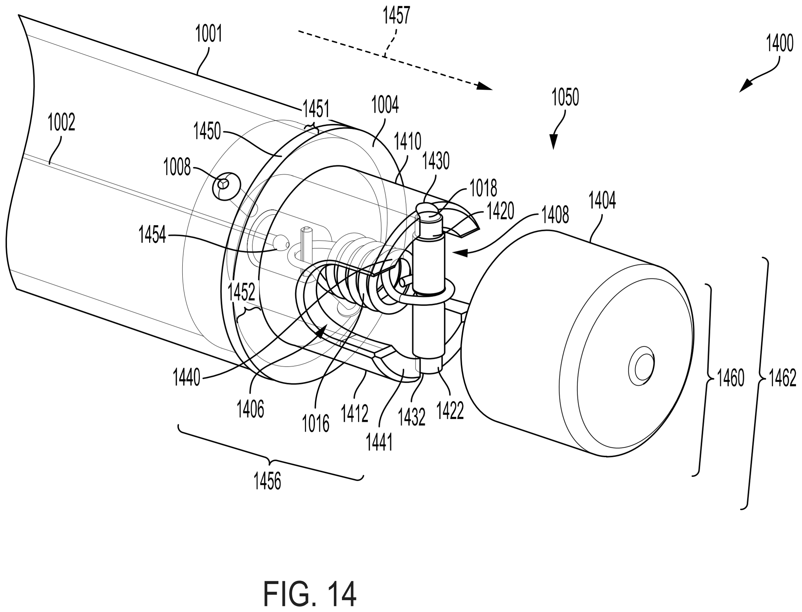

FIG. 12 shows a side view 1200 of first spherical swage-end termination 1012 seated in termination part 1015, which highlights the self-centering characteristic of wire 1002. Shank 1013 extends outward (to the left, in FIG. 12) from termination part 1015 in direction 1190. Because termination part 1015 is free-floating, meaning, coupled to spring 1016 via roll pin 1102 and not coupled directly to tube 1001 (via first end cap 1004), termination part 1015 is allowed to move to bring wire 1002 into a linear and perpendicular alignment with pin 1018 along a central axis 1202 of tube 1001, which may be secured to first end cap 1004 as shown in FIG. 14. As a result, tension applied to wire 1002 via spring 1016 (for example, when pin 1018 is secured to first end cap 1004 and/or first end cap 1004 is secured to tube 1001) may not cause a bend in wire 1002.

Termination part 1015 may comprise a first portion 1204 and a second portion 1206. First portion 1204 may be a cylindrical portion with a diameter 1212 and a length 1208. Second portion 1206 may be a mostly cylindrical portion with a length 1210 and a diameter 1212 but with a first recessed flat surface 1220 at a top side of termination part 1015 (in FIG. 12) and a second recessed flat surface 1222 at a bottom side of termination part 1015, with hole 1134 extending from first recessed flat surface 1220 to second recessed flat surface 1222. Roll pin 1102 may be inserted into hole 1134. A length 1213 of roll pin 1102 may be less than diameter 1212, but greater than a thickness 1214 of second portion 1206 measured between first recessed flat surface 1220 and second recessed flat surface 1222. When first spherical swage-end termination 1012 is seated in termination part 1015 and tension is applied to spring 1016, a spherical portion 1230 of first spherical swage-end termination 1012 may rest on lip 1140, with shank 1013 and cable 1002 extending out of first portion 1204 of termination part 1015.

FIG. 14 shows a second perspective view 1400 of first end 1050 of embodiment 1000 of wire phantom 302. Second perspective view 1400 shows a positioning of first end cap 1004 at first end 1050. First end cap 1004 may have a cylindrical shape including a first, flatter cylindrical portion 1450 that seals first end cap 1004 against tube 1001, and a second cylindrical portion 1454 that extends outward from tube 1001 in a direction indicated by an arrow 1457, towards first end 1050. First cylindrical portion 1450 may have a diameter 1462 that is equal to or greater than a diameter of tube 1001, and a height or thickness 1451 structurally sufficient to withstand a desired tension of wire 1002 without deforming or being damaged. The desired tension may be, for example, 15 lbf. Second cylindrical portion 1454 has a height 1456, which may be configured to enclose spring 1016 when spring 1016 is engaged to provide the desired tension of wire 1002. Second cylindrical portion 1454 has a diameter 1460 that is smaller than diameter 1462 by a distance 1452, such that first cylindrical portion 1450 extends circumferentially outward from second cylindrical portion 1454 by distance 1452. Second perspective view 1400 shows a cap 1404 that may have a height approximately equal to height 1456 of second cylindrical portion 1454 and an inner diameter equal to diameter 1460 of second cylindrical portion 1454, such that cap 1404 may slidably inserted over second cylindrical portion 1454 to cover and protect the coupling of wire 1002 to first end cap 1004 and provide a clean look. Cap 1404 may be made of plastic, in various examples.

First end cap 1004 includes cutaway sections 1406 and 1408 at opposing sides of termination part 1015, which may allow spring 1016 to be held or manipulated while fastening first end cap 1004 to tube 1001 (e.g., via fasteners 1008). Cutaway sections 1406 and 1408 also create saddle portions 1410 and 1412 between cutaway sections 1406 and 1408, which may support pin 1018 when tension is applied to spring 1016. Pin 1018 may include a first circumferential end portion 1420 with a reduced diameter that may be seated into a recessed portion 1430 of an outer surface 1440 of saddle portion 1410, and a second circumferential end portion 1422 with a reduced diameter that may be seated into a recessed portion 1432 of an outer surface 1441 of saddle portion 1412, which may prevent pin 1018 from moving laterally along outer surfaces 1440 and 1441 of saddle portions 1410 and 1412, respectively.

Referring now to FIG. 15, a perspective view 1500 shows second end 1052 of embodiment 1000 of wire phantom 302. Second perspective view 1500 shows a positioning of second end cap 1006 at second end 1052 (e.g., a base-end fitting). Second end cap 1006 has a diameter that is equal to or greater than diameter 1462 of tube 1001, such that second end cap 1006 seals tube 1001 at second end 1052. Second end cap 1006 may be coupled to a base plate 336, which may couple second end cap 1006 to phantom holder 323, as described above in reference to FIG. 3A. Wire 1002 may be coupled to second end cap 1006 via a bolt 1040, where second spherical swage-end termination 1014 may be seated in bolt 1040 in a similar manner as described above with respect to first end cap 1004. Bolt 1040 may be bolted to a circular inner surface 1502 of the second end cap, at a center point of circular inner surface 1502. A spherical head bolt 1520 may allow base plate 336 to pivot in an x/y dimension, as indicated by reference axes 301.

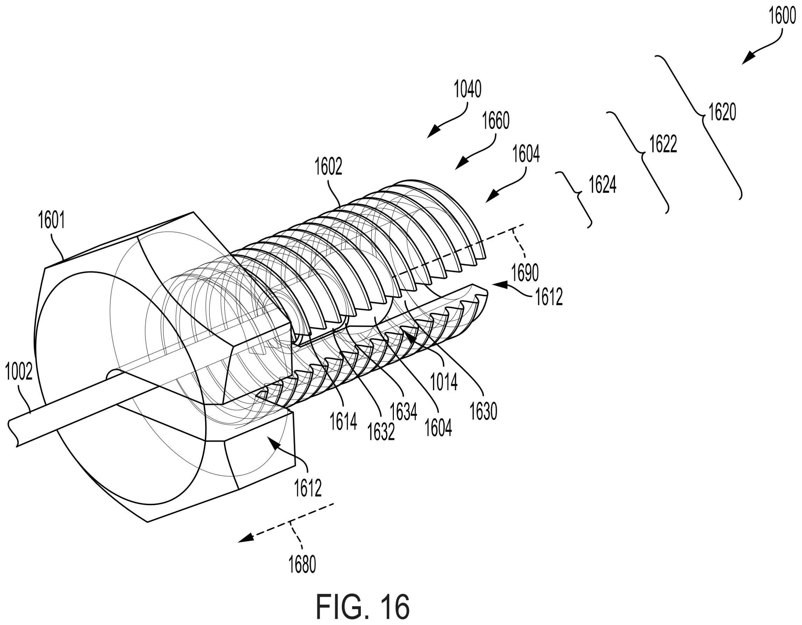

FIG. 16 shows a perspective view 1600 of bolt 1040, showing second spherical swage-end termination 1014 seated in bolt 1040. Bolt 1040 has a head portion 1601 and a threaded portion 1602 with a diameter 1620. Threaded portion 1602 may be hollow, and may include a first cylindrical chamber 1630 coaxially aligned around a central axis 1690 of bolt 1040. First cylindrical chamber 1630 may have a diameter 1622 (e.g., less than diameter 1620). Diameter 1622 may be greater than a diameter of a spherical portion 1604 of second spherical swage-end termination 1014. Threaded portion 1602 may include a second cylindrical chamber 1632 coaxially aligned around central axis 1690 that extends from first cylindrical chamber 1630 towards head portion 1601. Second cylindrical chamber 1632 may have a diameter 1624, which may be greater than a diameter of a shank 1614 of second spherical swage-end termination 1014, but less than the diameter of spherical portion 1604 of second spherical swage-end termination 1014. Both of head portion 1601 and threaded portion 1602 may include a slot 1612 that extends into a center of bolt 1040 and opens into first cylindrical chamber 1630 and second cylindrical chamber 1632, which allow wire 1002 to be inserted laterally into bolt 1040 as spherical portion 1604 is inserted into first cylindrical chamber 1630 at an end 1660 of threaded portion 1602. After spherical portion 1604 is inserted into first cylindrical chamber 1630, wire 1002 may be pulled (via spring 1016 at first end 1050 of tube 1001) in a direction indicated by an arrow 1680, which may seat second spherical swage-end termination 1014 within threaded portion 1602 as shank 1614 slides into second cylindrical chamber 1632. When second spherical swage-end termination 1014 is seated within threaded portion 1602, spherical portion 1604 may abut against a lip 1634 between first cylindrical chamber 1630 and second cylindrical chamber 1632, securing wire 1002 within bolt 1040. Bolt 1040 may then be coupled to second end cap 1006 as shown in FIG. 15.

FIG. 3C shows a detector array diagram 360 indicating an exemplary positioning of wire 304 with respect to the detector array during calibration using wire phantom 302. Detector array diagram 360 includes an x-ray source 362 and a detector array 364. X-ray source 362 and detector array 364 may be mounted at fixed positions on a gantry (e.g., gantry 102). X-ray source 362 and a center point 363 of detector array 364 may be aligned along a y axis of the CT imaging system, where detector array 364 and x-ray source 362 rotate around an isocenter 380 of the gantry. The gantry may rotate in a direction indicated by arrow 382.

Detector array 364 has a shape of an arc in a first dimension along an x axis of the CT imaging system, and may be flat in a second dimension along a z axis of the CT system. During a calibration scan, a table of the CT system (not shown in FIG. 3C) may be positioned along the z axis, where wire 304 may be coupled to an end of the table such that wire 304 extends between detector array 364 and x-ray source 362. Thus, a position of wire 304 relative to detector array 364 and x-ray source 362 changes as the gantry rotates.

In the position depicted in detector array diagram 360, an x-ray 372 directed at a detector element 368 may be attenuated by wire 304 when x-ray 372 strikes wire 304 at a point 370 on wire 304, whereby detector element 368 may detect less of x-ray 372. However, other x-rays emitted by x-ray source 362 at a same x position (e.g., along the x-dimension depicted by arrow 384) may be detected by other detector elements positioned at the same x position on detector array 364. Thus, the attenuation of x-ray 372 by wire 304 may be used to establish a linear relationship between detector element 368, point 370 on wire 304, and x-ray source 362. Once the linear relationship has been established, projection geometry may be leveraged to measure a position of detector element 368 on detector array 364 and an angle of alignment of detector element 368 in the x and z axes with respect to wire 304. How the position of detector element 368 is calculated based on the attenuation of x-ray 372 is explained in greater detail below in reference to FIGS. 7, 8, and 9.

Referring now to FIG. 7, a wire position graph 700 shows an exemplary line 702 indicating a position of a wire of a wire phantom, such as wire 304 of wire phantom 302 of FIG. 3A, as detected by a plurality of detector elements of a detector array, over a plurality of view angles, during one full rotation of a gantry during a scan performed by a CT imaging system. Frames corresponding to different view angles are shown on an x axis of wire position graph 700, and a pixel (e.g., detector element) position in an x dimension is shown on a y axis of wire position graph 700.

Line 702 has a shape of a sinogram, due to the rotation of the gantry around the wire, as described above. During calibration, the wire phantom may be coupled to a table at a fixed position with respect to the gantry. The fixed position may be outside a field of view (FOV) of the detector array, to ensure that all detector elements of the detector array are calibrated. For example, the fixed position may be 50 cm outside the FOV. In other words, if the fixed position were inside the FOV, some detector elements of the detector array between the fixed position and an edge of the FOV would not detect the wire, and would not be calibrated. As a result of the fixed position being outside the FOV, a top portion 750 of line 702 and a bottom portion 751 of line 702 may be excluded from wire position graph 700.

For example, a first point 704 on line 702 may indicate that at a view angle corresponding to a 200^{th} frame, a first attenuation of x-rays by the wire (referred to herein as wire attenuation) is detected to a greatest degree by a first detector element at an x position of 350 of a detector array including the detector element. The first wire attenuation may indicate the x position (e.g., 350) of the detector element within the detector array.

As a second example, a second point 705 on line 702 may indicate that at a view angle corresponding to a 1350^{th} frame, a second wire attenuation is detected to a greatest degree by the first detector element at the x position of 350 of the detector array. The second wire attenuation may confirm the x position (e.g., 350) of the detector element within the detector array. Each detector element of the detector array may detect the wire at two different frames of the full rotation.

Because a diameter of the wire is greater than a transaxial diameter of a detector element of the detector array, the attenuation of x-rays by the wire may be detected by more than one neighboring detector element. For example, if the diameter of the wire is one pixel, and the transaxial diameter of the detector element is 356 microns, 3-5 neighboring detector elements may record the attenuation. The attenuation may be strongest at a center pixel of the 3-5 neighboring detector elements, where line 702 corresponds to the center pixels recording the wire attenuation as the gantry rotates. The wire attenuation may be less at the neighboring detector elements, generating a wire shading 709 around line 702.

In wire position graph 700, wire shading 709 around line 702 may have a horizontal extent 710, where horizontal extent 710 is based on a number of view angles over which the attenuation is recorded by a single detector element. Wire shading 709 may also have a vertical extent 720, where vertical extent 720 is based on a number of pixels recording the attenuation at a given view angle.

For example, first dashed line 706 indicates that at the 200^{th} frame, x-rays are attenuated by the wire most at the detector element at the x position of 350 (on the y axis) corresponding to first point 704. However, x-rays are also attenuated to a lesser degree by neighboring detector elements in the x direction (on the y axis), such as detector elements corresponding to a first neighboring point 711 and a second neighboring point 713.

Similarly, a second dashed line 712 indicates that a single detector element may detect the attenuation of the wire over a plurality of neighboring frames/view angles. As the position of the detector element with respect to the wire changes due to the rotation of the gantry, the detector element may detect an attenuation that increases to a maximum attenuation and then decreases to zero. For example, second dashed line 712 indicates that a detector element at an x position of 1050 (on the y axis) may record a first, lower attenuation at a 1650^{th} frame corresponding to a point 718; a second, maximum attenuation at a 1700^{th} frame corresponding to a point 714 (e.g., on line 702); and a third, lower attenuation at a 1750^{th} frame corresponding to a point 719.

FIG. 8 shows a fitting diagram 800 that illustrates how an exact x position of a detector element within the detector array may be precisely determined from line 702, by fitting a function to measurements made at the detector element. For example, the function may be a Gaussian function, or a parabolic function, or a different kind of function. Fitting diagram 800 includes a wire position graph 802 similar to wire position graph 700, where an exemplary line 803 indicates a position of a wire of a wire phantom, in the shape of a sinogram, as generated by wire attenuation measurements recorded at a plurality of detector elements/pixels. An x position of pixels within the detector array is indicated along a y axis of graph 802, and a plurality of frames over which the gantry is rotated is indicated on the x axis, as in FIG. 7. A portion 812 of line 803 is shown in an expanded view 804, where portion 812 indicates wire shading around line 803.

A graph 806 shows a plot 820 of measurements 822 collected at a plurality of pixels corresponding to portion 812, centered around a pixel at an x position of 300 on the detector array, indicated by a dashed line 805. A strength of a signal generated at the pixels is shown on a y axis of graph 806, and the x position of the pixels is indicated on the x axis of graph 806, such that each measurement shown in graph 806 indicates a normalized signal strength between 0.0 and 1.0 recorded for a corresponding pixel. A lowest signal strength 824 is recorded for the pixel at the x position of 300 (e.g., 0.3), where x-rays are most attenuated by the wire. Low signal strength measurements at 828 and 829 (e.g., 0.4) indicate wire shading, where x-rays may be partially attenuated by the wire. Less shading is indicated at other neighboring pixels, but the shading does not exceed two pixels on either side of the pixel at the x position of 300.

To precisely determine a pixel location where the x-rays are most attenuated by the wire, a parabolic function 826 may be fitted to the measurements 822. Because a thickness (e.g., diameter) of the wire may be greater than a transaxial dimension of the detector elements (e.g., several times greater), more than one detector element may record high levels of attenuation by the wire. Parabolic function 826 may be used to select a single detector element (e.g., the pixel location) at which the wire attenuation is the greatest. By using a wire with a thickness greater than the transaxial dimension of the detector elements, an accuracy of the pixel location may be increased with respect to other wire phantoms that include a thinner wire. For example, an alternative wire phantom with a thinner wire may generate inconsistent and/or missing signals as an x-ray beam sweeps across a detector array. Alternatively, using a wire that is too thick may obscure too many detector elements, reducing the accuracy of the pixel location. In various embodiments, the wire thickness may be selected based on dimensions or properties of the CT system being calibrated. For example, in one embodiment, the selected thickness may be a function of a first distance between adjacent detector elements (e.g., detector pitch), a size of a focal spot of the CT system, and a second distance between the focal spot and the detector elements. In other embodiments, other or different variables may be considered.

An attenuated signal weighted average may not provide a needed accuracy of the centroid of a wire position due to measurement variability caused by limited x-ray count statistics. Exemplary graph 806 shows a parabolic function fit to reduce a statistical variability or measurement to measurement variation. Exemplary graph 806 shows a fitting in a pixel direction to calculate a pixel centroid, but the fitting can be done to a frame direction to get a frame, depending on the final fitting model.

Once the single detector element at which the wire attenuation is the greatest is selected, a position of the single detector element with respect to the wire (e.g., a known location of the wire) may be measured using projection geometry. Measurement of the position of the single detector is described in greater detail below in reference to FIGS. 5A, 5B, and 9.

FIG. 5A shows a flowchart illustrating an exemplary method 500 for using calibration data to increase a quality of images reconstructed using a CT system, such as CT imaging systems 100 and 200 described above. As described above, during calibration of the CT system, a calibration vector may be generated including measured positions (or correction values) for each detector element of each detector array of the CT imaging system (e.g., detector arrays 108 and 400). During a subsequent scan of a subject (e.g., a patient) using the CT system, the calibration vector may be applied during image reconstruction, to correct for misalignments of the detector elements. Specifically, a processing of the projection data during image reconstruction may rely on a precise position of each detector element. However, a design target position of the detector element, for example, provided by a detector design drawing based on physical dimensions and characteristics of the CT imaging system, may differ from an actual, measured position of the detector element. The measured positions may be used to reconstruct the image, rather than the design target positions. By correcting for the misalignments, an accuracy and image quality of the reconstructed images may be increased. Method 500 may be stored as instructions in a non-transitory memory and executed by one or more processors of a computing device of a CT imaging system, such as computing device 216 of imaging system 200 of FIG. 2.

Method 500 begins at 502, where method 500 includes performing a calibration of one or more detector arrays of the CT imaging system using a wire phantom. Performing the calibration of the one or more detector arrays using the wire phantom is described in greater detail in reference to FIG. 5B.

At 504, method 500 includes storing a calibration vector generated during calibration of the CT imaging system in a memory of the CT imaging system, such as in a memory of computing device 216 of FIG. 2. The calibration vector may be retrieved and applied during processing of projection data acquired via the CT imaging system, until a new calibration vector is generated during a subsequent calibration.

At 506, method 500 includes performing a scan on a subject. During the scan, projection data may be acquired by the CT imaging system. However, the projection data may include pixel-level inaccuracies, due to misalignment of detector elements (e.g., detector elements 202) positioned on detector arrays of the CT imaging system.

At 508, method 500 includes applying the calibration vector during processing of the acquired projection data during image reconstruction. The calibration vector may include measured positions of each detector element in a detector array. During the image reconstruction, a signal generated by each detector element is processed based on a design target position of the detector element within a detector array. If an actual (e.g., measured) position of the detector element within the detector array is different from the design target position, an image quality of a resulting image may be reduced. Therefore, the measured positions of the detector elements stored in the calibration vector may be used instead of the design target positions.

Alternatively, in some embodiments, the calibration vector may include alignment correction values to correct for misalignments of the detector elements, where each position of each detector element in the projection data may be adjusted by applying a corresponding position correction value of the calibration vector. It should be appreciated that the calibration vector referred to herein should not be confused with other, different calibration vectors that may additionally be applied, for example, to correct for variations in signals from (correctly aligned) detector elements.

At 510, method 500 includes displaying a reconstructed image on a display device of the CT imaging system (e.g., display device 232 of FIG. 2) and/or storing the reconstructed image in a memory of the CT imaging system, and method 500 ends.

FIG. 5B shows a method 550 illustrating an exemplary procedure for calibrating a detector array of the CT imaging system using the wire phantom. Various steps of method 550 may be stored as instructions in a non-transitory memory and executed by one or more processors of a computing device of a CT imaging system, such as computing device 216 of imaging system 200 of FIG. 2. Some steps of method 550 may not be performed by the computing device, and may be performed manually, as indicated below.

Method 550 begins at 552, where method 550 includes installing the wire phantom at a fixed position on a table of the CT imaging system (e.g., table 114). The wire phantom may be installed manually, for example, by an operator of the CT imaging system. The wire phantom may be installed as described in relation to FIG. 3B, where the wire phantom may be mounted on an end or edge of the table, such that a wire of the wire phantom (e.g., wire 304) extends into a space between the detector array and an x-ray source, as described above in relation to FIG. 3C. The fixed position of the wire may be outside a field of view of the detector array. As an example, the wire phantom may be mounted on the table such that the wire is positioned at 180 degrees and 27cm below the gantry iso center using a gantry horizontal and vertical laser. A level may be used on the wire tube to make it as level as possible with a Z axis of the CT imaging system. A center of the wire may be indicated as a 0 mm reference position in Z.

In some examples, method 550 may include providing instructions to the operator to move the table to a specified height and/or to a specified position along a z-axis of the CT imaging system. For example, after the operator mounts the wire phantom on the end or edge of the table, the instructions may be provided via a display device of the CT imaging system (e.g., display device 232).

At 554, method 550 includes determining whether the wire is verified to be straight, e.g., within a threshold tolerance for straightness. In various embodiments, the wire may be verified to be straight based on a received input of the operator. If there are variations in the straightness, the wire may not be used for calibrating the detector array. In one example, the threshold tolerance is 30 microns, where if a portion of the wire deviates from straight by more than 30 microns, the wire may not be considered straight, and may not be used for calibrating the detector array. If the wire does not deviate from straight by more than 30 microns, the wire may be verified as straight, and may be used for calibrating the detector array. If the wire phantom is new, or has not been tested recently, the straightness of the wire may be verified prior to continuing with the calibration. For example, the straightness of the wire may be periodically verified, such as every six months, or after a predetermined number of scans (e.g., 600) have been performed.

If at 554 it is determined that the wire has not been verified to be straight, method 550 proceeds to 556. At 556, method 550 includes performing an analysis of the straightness of the wire. During the straightness analysis, rotational scans of the wire phantom may be performed with the wire phantom at different positions along the z axis. In other words, between each rotational scan, a position of the wire phantom along the z axis may be adjusted by small increments, such as 5 mm. During each rotational scan, data may be collected from a small number (e.g., 2-4) of center rows of the detector array, and data may not be collected from other rows of the detector array. The data collected from the small number of center rows may be used to determine whether the wire is within the threshold tolerance for straightness.

For example, with the wire phantom mounted on the table, a position of the table along the z axis may be adjusted such that a plane of rotation of the gantry in the x-y dimension (e.g., an imaging plane) intersects with the wire of the wire phantom at a first point on the wire. The plane of rotation may include a line along a y axis of the CT imaging system (e.g., dashed line 420 of FIG. 4) between an x-ray source of the CT imaging system and a center point of the detector array (e.g., x-ray source 362 and center point 363 of FIG. 3C), where the line is perpendicular to the z axis. A first rotational scan may be performed, and a first set of wire attenuation data from the 2-4 center rows of the detector array may be collected. The position of the table may then be adjusted by an increment to a second specific point, and a second rotational scan may be performed, and a second set of wire attenuation data from the 2-4 center rows of the detector array may be collected. The position of the table may then be adjusted by the increment to a third specific point, and a third rotational scan may be performed, and a third set of wire attenuation data may be collected, and so on, until data has been collected at a desired plurality of wire positions along the z axis.

For example, the table may be adjusted such that the imaging plane of the gantry intersects with the wire at a position of -55 mm with respect to the 0 position at the center of the wire, and the table may be adjusted by 5 mm increments along the z axis until reaching a position of 55 mm with respect to the 0 position, for a total of 23 scans. The 55 mm may be sufficient to cover a Z axis imaging field-of-view.

Referring briefly to FIG. 6, a wire phantom alignment diagram 600 shows a positioning of the wire phantom 605 at three different times during a collection of wire attenuation data, according to one embodiment. Wire phantom 605 is coupled to a table mount 606 (e.g., table mount 320), which is coupled to a table 608 (e.g., table 114).

At a first time 601, wire phantom 605 has been positioned such that an x-y rotational plane of the gantry indicated by a dotted line 607 intersects with a wire 604 of wire phantom 605 at a first point 614 of wire 604. The imaging plane of the gantry may be perpendicular to the z axis, shown by an arrow 650 and reference axes 690, and may include an x-ray source 620 on a first side of the gantry, a center point 612 of a detector array 610 positioned on a second, opposite side of the gantry, and an isocenter point 611 around which the gantry rotates. Wire phantom 605 is offset from isocenter point 611, such that as x-ray source 620 and detector array 610 rotate around isocenter point 611, a distance 615 between wire 604 and detector array 610 varies. First point 614 may be a center point of wire 604, and first point 614 may be landmarked as a 0 mm reference point of wire 604. At first time 601, wire 604 is positioned within a FOV of detector array 610.

At a second time 602, the position of wire phantom 605 along the z axis has been shifted in a first, positive direction indicated by a first arrow 622. Wire phantom 605 has been positioned such that the imaging plane indicated by dotted line 607 intersects with wire 604 of wire phantom 605 at a second point 616 of wire 604. Second point 616 may be at a 55 mm reference point of wire 604 (e.g., 55 mm from first point 614 at 0 mm, in the positive direction). At second time 602, wire 604 is positioned partially outside the FOV of detector array 610.

At a third time 603, the position of wire phantom 605 along the z axis has been shifted in a second, negative direction indicated by a second arrow 624. Wire phantom 605 has been positioned such that the imaging plane indicated by dotted line 607 intersects with wire 604 of wire phantom 605 at a third point 618 of wire 604. Third point 618 may be at a -55 mm reference point of wire 604 (e.g., 55 mm from first point 614, in the negative direction). At third time 603, wire 604 is positioned partially outside the FOV of detector array 610.

To determine a straightness of wire 604, data may be collected at a plurality of increments between first point 614 and third point 618, such as 5mm increments, as described above. By collecting and comparing the data at the plurality of increments, a straightness of wire 604 may be precisely determined. For example, if a first set of wire attenuation data collected at second time 602 indicates a first position of a first portion of wire 604, and a second set of wire attenuation data collected at third time 603 indicates a second position of a second portion of wire 604, a deviation between the first position and the second position may indicate the first portion is not aligned with the second portion, whereby it may be inferred that wire 604 is not straight. If the deviation is less than a threshold deviation (e.g., 30 microns), wire 604 may be considered straight.

Returning to method 550, at 558, method 550 includes determining whether the wire is straight, in accordance with the procedure described above in reference to FIG. 6. If at 558 it is determined that the wire is not straight, method 550 proceeds to 560. At 560, method 550 includes displaying instructions on the display device to the operator for fixing or replacing the wire, and method 550 proceeds back to 556. If at 558 it is determined that the wire is straight, method 550 proceeds to 562.

At 562, method 550 includes performing an angle alignment analysis of the wire to determine an alignment of the wire with the z axis of the CT imaging system, along which the table is aligned. In one example, if the wire deviates from parallel with the z axis by less than a threshold deviation, the wire is considered to be aligned with the z axis. For example, the threshold deviation may be 3 mrad. In other words, if the wire deviates from parallel with the z axis by 3 mrads or more, the wire may be adjusted using adjustment tilt screws (e.g., the tilt screws 326 and 332 of FIG 3A), and the alignment data collection and analysis may be repeated until less than 3 mrad is achieved, as described in the next paragraph.

To perform the wire alignment analysis, a second set of rotational scans may be performed. The second set of rotational scans may be performed in a similar manner as described above in relation to FIG. 6. However, a fewer number of scans may be performed since the wire is known to be straight. In one embodiment, two wire alignment scans may be performed. A first wire alignment scan of the wire phantom may be performed with the wire phantom positioned such that the imaging plane intersects with the wire at a first distance from the center, reference point of the wire (e.g., at 0 mm) in a first direction (e.g., arrow 622 of FIG. 6). A second wire alignment scan of the wire phantom may be performed with the wire phantom positioned such that the imaging plane intersects with the wire at a second distance from the center, reference point of the wire in a second, opposite direction (e.g., arrow 624). The second distance may be equal to the first distance. The first distance and the second distance may be separated by a threshold distance. For example, the threshold distance may be 80 mm.

For example, the first distance and the second distance may both be 40 mm. The table may be first adjusted to position the wire such that the imaging plane intersects with the wire at 40 mm with respect to the center, reference point on the wire, to perform a first rotational scan. The table may be subsequently adjusted to position the wire such that the imaging plane intersects with the wire at -40 mm with respect to the center, reference point on the wire, to perform a second rotational scan. At each of the first and the second rotational scans, wire attenuation data may be acquired at the 2-4 center rows of detector elements of the detector array. Performing the angle alignment analysis may be faster than performing the straightness analysis, as the wire is already verified to be straight.

By collecting and comparing the data from the first rotational scan and the second rotational scan, an angular alignment of wire 604 with the z axis may be precisely determined. For example, if a first set of wire attenuation data collected during the first rotational scan indicates a first position of wire 604, and a second set of wire attenuation data collected during the second rotational scan indicates a second position of wire 604, a deviation between the first position and the second position may indicate that the wire is out of alignment. If the deviation is less than the threshold deviation (e.g., 3 mrad), the wire may be considered aligned with the z axis.

At 564, method 550 includes determining whether the wire is aligned with the z axis, in accordance with the procedure described above. If the wire is determined not to be aligned with the z axis (e.g., with a deviation greater than the threshold deviation), method 550 proceeds to 566. At 566, method 550 includes displaying instructions on the display device for realigning the wire. In various embodiments, realigning the wire may be performed by the operator by adjusting one or more tilt screws of the wire phantom. For example, a vertical tilt screw positioned on a phantom holder of the wire phantom (e.g., tilt screw 326 of phantom holder 323) may adjust the wire relative to the y axis, and a horizontal tilt screw (e.g., tilt screw 332 of phantom holder 323) may adjust the wire relative to the x axis. After the wire has been realigned, method 550 proceeds back to 562, where the wire angle alignment analysis may be performed again. In some cases, the wire may be realigned and the wire angle alignment analysis may be performed a plurality of times to ensure that the wire is aligned with the z axis.

If at 564 the wire is determined to be aligned with the z axis, method 550 proceeds to 568. At 568, method 550 includes performing a calibration scan to measure a precise position of each individual detector element (detector elements 202) of the detector array with respect to the wire. Since the position of the wire is known and fixed, the precise position of each individual detector element may be measured by performing a full rotation and fitting a function to the sinogram generated by the projection data, as described above in reference to FIGS. 7 and 8, and using projection geometry based on physical characteristics of the CT system.

Referring to FIG. 9, a projection geometry diagram 900 illustrates a plurality of variables used to calculate the position of the wire. Projection geometry diagram 900 shows an exemplary CT system 902 (e.g., CT system 100 or imaging system 200) including an x-ray source 904 and a detector array 906, which may be a non-limiting example of detector array 400 of FIG. 4. X-ray source 904 and detector array 906 may be coupled to a gantry (e.g., gantry 102) that rotates in a direction 901 around an isocenter (point) 914, where isocenter 914 is half way between x-ray source 904 and a center point 907 of detector array 906, along an isocenter line 905. Detector array 906 includes a plurality of detector modules 908, where each detector module 908 includes a plurality of pixels 912. The pixels 912 may be organized in slices or chiclets 910. Each pixel 912 of detector array 906 may be referenced by a distance 909 from center point 907 along detector array 906 in an x dimension, and a row of the pixel 912 in a z dimension, based on references axes 990. A wire of a wire phantom is positioned within CT system 902 at a wire location 916.

FIG. 9 depicts the CT system at a point in time when the gantry has been rotated from an initial position, indicated by a line 926, by an angle of rotation 922 (β) of the gantry with respect to line 926. Wire location 916 (in reference to x-ray source 904 and/or detector array 906) may be measured based on distance R between wire location 916 and isocenter 914, indicated by a line 917, and an angle 918 (φ) between line 917 and line 926.

At the point in time depicted in FIG. 9, x-ray beams emitted by x-ray source 904 are detected at detector array 906. However, a pixel 920 may not detect an x-ray beam on a trajectory indicated by a line 919, which impinges on the wire at wire location 916. Line 919 is at an angle 924 (γ) with isocenter line 905. Thus, the angle location of pixel 920, y, with respect to isocenter line 905 can be expressed as a function of a gantry rotation angle β based on *R* and φ, where R and φ are a measured wire position from the wire alignment step depicted previously. R is the wire distance from the isocenter, and φ is the wire angle from the isocenter line 905. SID is a Source-904 to-Isocenter 914 Distance, which is known by an engineering design. *β* 922 is a gantry rotation angle with respect to a gantry vertical Y axis 926. $\begin{matrix}γ \left(β\right) = {tan}^{- 1} \left(\frac{R sin α}{SID + R cos α}\right) , \\ α = φ - π - β\end{matrix}$ (1)

A design target location of pixel 920 may be provided by a parameterized detector model, as follows: ${γ}_{model} \left(X\right) = s \left(X\right) / SDD$ (2) where X represents a design model parameter; a design center location of each module and a pixel pitch within the module, from which a design location *γ*_{model} of each pixel is calculated and modeled, and SDD is a Source-to-Detector-Distance between 904 and 907 that is known by design. The equations (1) and (2) are numerically inversed to include all pixels, p, in a minimization optimization between a measured and design (model) pixel location: ${X}^{∗} = argmin {\left‖{β}_{meas}\left(p\right)-{β}_{model}\left(p\right)\right‖}_{X , R , φ} ,$ (3) As a result of a measurement described in FIG. 8, *p* vs *β* (pixel vs. view angle), is used to inverse equation (1). *X** represents the fitting result of a module location and pixel pitch, from which all pixel locations may be extracted.

At 570, method 500 includes optionally generating alignment correction values from the measured positions of the detector elements, where the alignment correction values may be based on differences between the measured positions and design target positions of the detector elements.

At 572, method 500 includes storing the measured positions of the detector elements (or the alignment correction values) in a calibration vector for use in a subsequent scan of a subject, and method 550 ends. In other words, a calibration vector may be generated that includes a measured position of each detector element, or a correction value indicating an adjustment to be made to a position of the detector element to correct for the detected and quantified misalignment. The calibration vector may be used by method 500 of FIG. 5A to increase a quality of a reconstructed image of a subject during a subsequent scan, as described above.

Thus, a method is provided for correcting for misalignments in detector elements of a detector array of a CT system. The method may be used when mechanical calibration techniques may not be feasible, due to a size of the detector elements. The method relies on a wire phantom including a wire that is thicker than a detector element, unlike other wire phantoms, which may result in inconsistent detector readings. In a first step, the wire phantom is mounted on a table of the CT system, and a straightness and an angle of inclination of the wire are verified in accordance with procedures described herein. Once the straightness and correct alignment of the wire has been verified, and a position of each detector element of the detector array may be measured with respect to the wire. The measured positions may be stored in a calibration vector and applied during a subsequent scan of a subject, during image reconstruction, to correct for the misalignments.

By using the measured positions of the detector elements rather than design target positions from an engineering model of the detector array, the misalignments of the detector elements may be corrected during scans on subjects, and an image quality of resulting reconstructed images may be increased. An advantage of using the measured detector element positions is that mechanical alignment techniques may not be relied on to adjust the alignment of the detector elements. As a result, the method may be applied to detector arrays including smaller detector elements than can be aligned using the mechanical alignment techniques. Additionally, a calibration time, resource allocation, and cost of the CT system may be reduced, making the CT system more efficient. Thus, a functioning of the CT system may be improved, leading to reduced down time and faster and more accurate processing during calibration using the CT system. Further, higher quality images may be generated by the CT system as a result of applying the calibration method described herein, which may result in a higher percentage of successful diagnoses and desirable patient outcomes.

The technical effect of using a wire phantom to calibrate positions of detector elements within a detector array of a CT system is that a quality of an image reconstructed using the CT system may be increased.

The disclosure also provides support for a wire phantom assembly for calibrating a computed tomography (CT) system, the wire phantom assembly comprising: a wire phantom including a wire mounted in a tube, and a phantom holder configured to support the wire phantom, the phantom holder mountable on a table of the CT system, wherein a straightness of the wire in the tube is maintained by a tension on the wire between a first spherical swage-end termination of the wire coupled to a first end of the wire phantom, and a second spherical swage-end termination coupled to a second, opposite end of the wire phantom. In a first example of the system, the first spherical swage-end termination is seated in a free-floating termination part that is coupled to a first end cap of the tube at the first end via a spring, the spring generating the tension. In a second example of the system, optionally including the first example, a first end coil of the spring is coupled to the termination part via a first pin extending through a hole in the termination part. In a third example of the system, optionally including one or both of the first and second examples, the first end cap includes a first cylindrical portion having a first diameter equal to or greater than a diameter of the tube that seals the first end cap against the tube, and a second cylindrical portion having a second diameter and cutaway sections on opposing sides of the second cylindrical portion that create saddle portions of the second cylindrical portion between the cutaway sections. In a fourth example of the system, optionally including one or more or each of the first through third examples opposing end coil of the spring is coupled to the first end cap via a second pin, a first side of the second pin seated in a first recessed portion of a first outer surface of a first saddle portion of the first end cap, and a second side of the second pin seated in a second recessed portion of a second outer surface of a second saddle portion of the first end cap. In a fifth example of the system, optionally including one or more or each of the first through fourth examples, the second pin has a first circumferential end portion with a reduced diameter that is seated in the first recessed portion, and a second circumferential end portion with a reduced diameter that is seated in the second recessed portion. In a sixth example of the system, optionally including one or more or each of the first through fifth examples, the system further comprises: a cylindrical cap having an inner diameter equal to the diameter of the second cylindrical portion that is slidably inserted over the second cylindrical portion to cover and protect a coupling of the wire to the first end cap. In a seventh example of the system, optionally including one or more or each of the first through sixth examples, the second spherical swage-end termination is seated in a hollow threaded portion of a bolt that is bolted to a center point of an inner circular surface of a second end cap of the tube at the second end of the tube. In a eighth example of the system, optionally including one or more or each of the first through seventh examples, the hollow threaded portion of the bolt includes a first cylindrical chamber coaxially aligned around a central axis of the bolt with a first diameter greater than a diameter of a spherical portion of second spherical swage-end termination, and a second cylindrical chamber coaxially aligned around the central axis that extends from the first cylindrical chamber towards a head portion of the bolt, the second cylindrical chamber having a second diameter that is greater than a diameter of a shank of the second spherical swage-end termination, but less than the diameter of the spherical portion. In a ninth example of the system, optionally including one or more or each of the first through eighth examples, both of the head portion and the hollow threaded portion of the bolt include a slot that extends into a center of the bolt, opening into the first cylindrical chamber and the second cylindrical chamber, which allows the wire to be inserted laterally into the bolt as the spherical portion of the second spherical swage-end termination is inserted into the first cylindrical chamber at an end of the threaded portion, with the shank inserting into the second cylindrical chamber, such that the second spherical swage-end termination abuts against a lip between the first cylindrical chamber and the second cylindrical chamber to secure the wire within the bolt when the tension is applied to the wire. In a tenth example of the system, optionally including one or more or each of the first through ninth examples, the wire is a braided stainless steel cable. In a eleventh example of the system, optionally including one or more or each of the first through tenth examples, the phantom holder is configured to be secured to the table via a clamp coupled to a cradle attached to a back surface of the phantom holder, the clamp including a knob and a threaded portion that is inserted through a hole in a bearing plate coupled to a top edge of the phantom holder that when rotated via the knob exerts a force on a lower portion of the cradle that increases a pressure between a bottom surface of the lower portion and an upper edge of the table. In a twelfth example of the system, optionally including one or more or each of the first through eleventh examples, a flange portion of the cradle is bolted to the back surface via a plurality of bolts, and the bearing plate is coupled to the top edge of the phantom holder via a plurality of screws. In a thirteenth example of the system, optionally including one or more or each of the first through twelfth examples, the cradle is manufactured from a material that is flexibly deformed under pressure, and the pressure between the bottom surface of the lower portion and the upper edge of the table is generated as a result of the cradle flexibly deforming due to the force exerted by the threaded portion of the clamp. In a fourteenth example of the system, optionally including one or more or each of the first through thirteenth examples, the bearing plate is extended to include a handle portion including a hole into which a person's hand may be inserted, the handle portion configured to allow wire phantom assembly to be manually moved, carried, or mounted on the table, the hole positioned on an opposite side of the wire phantom assembly from the clamp and the cradle with a lift point of the wire phantom assembly close to a center of gravity of wire phantom assembly such that the wire phantom assembly remains level when lifted by handle portion.

The disclosure also provides support for a computed tomography (CT) system, comprising a processor and a non-transitory memory including instructions that when executed, cause the processor to: perform a first series of rotational scans of a wire phantom to determine a straightness of a wire of the wire phantom, perform a second series of rotational scans of the wire phantom to determine an angle of inclination of the wire, in response to the straightness of the wire being within a first threshold deviation and the angle of inclination of the wire being within a second threshold deviation: during a calibration scan performed using the CT system: measure a position of each detector element of a detector array of the CT system with respect to a location of the wire, and during a subsequent scan performed using the CT system: apply the measured position of each detector element during reconstruction of an image from projection data acquired via the CT system, and display the reconstructed image on a display device of the CT system, wherein the straightness of the wire is maintained within the first threshold deviation by a tension on the wire between a first spherical swage-end termination of the wire seated in a free-floating termination part coupled to a first end cap at a first end of a tube of the wire phantom via a spring, and a second spherical swage-end termination seated in a hollow threaded portion of a bolt bolted to center point of an inner circular surface of a second end cap of the tube at a second, opposite end of the wire phantom. In a first example of the system, the phantom holder is configured to be secured to the table via a clamp coupled to a cradle attached to a back surface of the phantom holder, the clamp including a knob and a threaded portion that is inserted through a hole in a bearing plate coupled to a top edge of the phantom holder that when rotated via the knob exerts a force on a lower portion of the cradle that increases a pressure between a bottom surface of the lower portion and an upper edge of the table, the pressure generated as a result of the cradle flexibly deforming due to the force exerted by the threaded portion of the clamp. In a second example of the system, optionally including the first example, the bearing plate is extended to include a handle portion including a hole into which a person's hand may be inserted, the handle portion configured to allow wire phantom assembly to be manually moved, carried, or mounted on the table, the hole positioned on an opposite side of the wire phantom assembly from the clamp and the cradle with a lift point of the wire phantom assembly close to a center of gravity of wire phantom assembly such that the wire phantom assembly remains level when lifted by handle portion.

The disclosure also provides support for a method for a computed tomography (CT) system, comprising: during a calibration of the CT system: measuring a position of a detector element of a detector array of the CT system using a wire of a wire phantom coupled to a table of the CT system, during a rotational scan performed using the CT system, during a subsequent scan performed on a subject using the CT system: applying the measured position of the detector element rather than a design target position of the detector element during reconstruction of an image from projection data acquired via the CT system, and displaying the reconstructed image on a display device of the CT system, wherein: the wire is maintained straight and centered in a tube of the wire phantom via a first spherical swage-end termination of the wire coupled to a free-floating termination part coupled to a first end cap of the tube via a spring that provides tension, and a second spherical swage-end termination seated in a hollow bolt coupled to a center point of a second, opposing end cap of the tube. In a first example of the method, the wire phantom is clamped to the table via a clamp coupled to a cradle attached to a phantom holder of the wire phantom, the clamp including a knob and a threaded portion that is inserted through a hole in a bearing plate of the phantom holder that when rotated via the knob, exerts a force on the cradle that increases a pressure between a bottom surface of the cradle and an upper edge of the table.

When introducing elements of various embodiments of the present disclosure, the articles "a," "an," and "the" are intended to mean that there are one or more of the elements. The terms "first," "second," and the like, do not denote any order, quantity, or importance, but rather are used to distinguish one element from another. The terms "comprising," "including," and "having" are intended to be inclusive and mean that there may be additional elements other than the listed elements. As the terms "connected to," "coupled to," etc. are used herein, one object (e.g., a material, element, structure, member, etc.) can be connected to or coupled to another object regardless of whether the one object is directly connected or coupled to the other object or whether there are one or more intervening objects between the one object and the other object. In addition, it should be understood that references to "one embodiment" or "an embodiment" of the present disclosure are not intended to be interpreted as excluding the existence of additional embodiments that also incorporate the recited features.

In addition to any previously indicated modification, numerous other variations and alternative arrangements may be devised by those skilled in the art without departing from the spirit and scope of this description, and appended claims are intended to cover such modifications and arrangements. Thus, while the information has been described above with particularity and detail in connection with what is presently deemed to be the most practical and preferred aspects, it will be apparent to those of ordinary skill in the art that numerous modifications, including, but not limited to, form, function, manner of operation and use may be made without departing from the principles and concepts set forth herein. Also, as used herein, the examples and embodiments, in all respects, are meant to be illustrative and should not be construed to be limiting in any manner.

## Claims

1. A wire phantom assembly (300) for calibrating a computed tomography (CT) system (100), the wire phantom assembly (300) comprising:
a wire phantom (302) including a wire (304) mounted in a tube (306); and
a phantom holder (323) configured to support the wire phantom (302), the phantom holder (323) mountable on a table of the CT system (100);
wherein a straightness of the wire (304) in the tube (306) is maintained by a tension on the wire (304) between a first spherical swage-end termination (1012) of the wire (304) coupled to a first end of the wire phantom (302), and a second spherical swage-end termination (1014) coupled to a second, opposite end of the wire phantom (302).

2. The wire phantom assembly (300) of claim 1, wherein the first spherical swage-end termination (1012) is seated in a free-floating termination part (1015) that is coupled to a first end cap (308) of the tube (306) at the first end via a spring (309), the spring (309) generating the tension.

3. The wire phantom assembly (300) of claim 2, wherein a first end coil (1014) of the spring (309) is coupled to the termination part (1015) via a first pin extending through a hole in the termination part (1015).

4. The wire phantom assembly (300) of claim 2, wherein the first end cap (308) includes a first cylindrical portion (1450) having a first diameter equal to or greater than a diameter of the tube (306) that seals the first end cap (308) against the tube (306) , and a second cylindrical portion (1454) having a second diameter and cutaway sections (1406) on opposing sides of the second cylindrical portion (1454) that create saddle portions (1410) of the second cylindrical portion (1454) between the cutaway sections (1406).

5. The wire phantom assembly (300) of claim 4, wherein a second, opposing end coil of the spring (309) is coupled to the first end cap (308) via a second pin, a first side of the second pin seated in a first recessed portion of a first outer surface of a first saddle portion of the first end cap (308), and a second side of the second pin seated in a second recessed portion of a second outer surface of a second saddle portion of the first end cap (308).

6. The wire phantom assembly (300) of claim 5, wherein the second pin has a first circumferential end portion (1420) with a reduced diameter that is seated in the first recessed portion, and a second circumferential end portion (1422) with a reduced diameter that is seated in the second recessed portion.

7. The wire phantom assembly (300) of claim 4, further comprising a cylindrical cap (1404) having an inner diameter equal to the diameter of the second cylindrical portion (1454) that is slidably inserted over the second cylindrical portion (1454) to cover and protect a coupling of the wire (304) to the first end cap (308).

8. The wire phantom assembly (300) of claim 1, wherein the second spherical swage-end termination (1014) is seated in a hollow threaded portion (1602) of a bolt (1040) that is bolted to a center point of an inner circular surface of a second end cap (312) of the tube (306) at the second end of the tube (306).

9. The wire phantom assembly (300) of claim 8, wherein the hollow threaded portion (1602) of the bolt (1040) includes a first cylindrical chamber (1630) coaxially aligned around a central axis (1690) of the bolt (1040) with a first diameter (1622) greater than a diameter of a spherical portion (1604) of second spherical swage-end termination (1014), and a second cylindrical chamber (1632) coaxially aligned around the central axis (1690) that extends from the first cylindrical chamber (1630) towards a head portion (1601) of the bolt (1040), the second cylindrical chamber (1632) having a second diameter (1624) that is greater than a diameter of a shank (1614) of the second spherical swage-end termination (1014), but less than the diameter of the spherical portion (1604).

10. The wire phantom assembly (300) of claim 9, wherein both of the head portion (1601) and the hollow threaded portion (1602) of the bolt (1014) include a slot that extends into a center of the bolt (1014), opening into the first cylindrical chamber (1630) and the second cylindrical chamber (1632), which allows the wire (304) to be inserted laterally into the bolt (1014) as the spherical portion (1604) of the second spherical swage-end termination (1014) is inserted into the first cylindrical chamber (1630) at an end of the threaded portion (1602), with the shank (1614) inserting into the second cylindrical chamber (1632), such that the second spherical swage-end termination (1014) abuts against a lip (1140) between the first cylindrical chamber (1630)and the second cylindrical chamber (1632) to secure the wire (304) within the bolt (1040) when the tension is applied to the wire (304).

11. The wire phantom assembly (300) of claim 1, wherein the wire (304) is a braided stainless steel cable.

12. The wire phantom assembly (300) of claim 1, wherein the phantom holder (323) is configured to be secured to the table via a clamp (1701) coupled to a cradle (1706) attached to a back surface (1722) of the phantom holder (323), the clamp (1701) including a knob (1702) and a threaded portion (1704) that is inserted through a hole in a bearing plate (1708) coupled to a top edge of the phantom holder (323) that when rotated via the knob (1702) exerts a force on a lower portion (1730) of the cradle (1706) that increases a pressure between a bottom surface (1738) of the lower portion (1730) and an upper edge of the table.

13. The wire phantom assembly (300) of claim 12, wherein a flange portion (1732) of the cradle (1706) is bolted to the back surface (1722) via a plurality of bolts, and the bearing plate (1708) is coupled to the top edge of the phantom holder (323) via a plurality of screws (1901, 1902, 1903).

14. The wire phantom assembly (300) of claim 13, wherein the cradle (1706) is manufactured from a material that is flexibly deformed under pressure, and the pressure between the bottom surface of the lower portion and the upper edge of the table is generated as a result of the cradle (1706) flexibly deforming due to the force exerted by the threaded portion (1704) of the clamp (1701).

15. The wire phantom assembly (300) of claim 12, wherein the bearing plate (1708) is extended to include a handle portion (2002) including a hole (2004) into which a person's hand may be inserted, the handle portion (2002) configured to allow wire phantom assembly (300) to be manually moved, carried, or mounted on the table, the hole (2004) positioned on an opposite side of the wire phantom assembly (300) from the clamp (1701) and the cradle (1706) with a lift point of the wire phantom assembly (300) close to a center of gravity of wire phantom assembly (300) such that the wire phantom assembly (300) remains level when lifted by handle portion (2002).
